(19) Europäisches Patentamt
European Patent Office
Office européen des brevets

(11) **EP 3 058 873 A1**

(12) **EUROPEAN PATENT APPLICATION**
published in accordance with Art. 153(4) EPC

(43) Date of publication:
**24.08.2016 Bulletin 2016/34**

(51) Int Cl.:
***A61B 5/16*** (2006.01)    ***A61B 3/11*** (2006.01)

(21) Application number: **14854821.7**

(22) Date of filing: **16.10.2014**

(86) International application number:
**PCT/JP2014/077559**

(87) International publication number:
**WO 2015/056742 (23.04.2015 Gazette 2015/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.10.2013  JP 2013216642**

(71) Applicant: **Natsume Research Institute, Co., Ltd. Tokyo 134-0087 (JP)**

(72) Inventors:
• **KIKUCHI, Kouichi**
  **Tokyo 134-0087 (JP)**
• **KURASHIMA, Wataru**
  **Tokyo 168-0082 (JP)**

(74) Representative: **Miller Sturt Kenyon**
  **9 John Street**
  **London WC1N 2ES (GB)**

(54) **DEVICE FOR MEASURING VISUAL EFFICACY**

(57)    An object, such as content to be viewed by a human, is further correctly evaluated. A visual object efficacy measuring device includes an analysis unit configured to calculate the degree of attention and the degree of feeling with respect to an object by analyzing an eyeball image and a face image of at least one viewer, and a determination unit configured to calculate visual object efficacy as an evaluation value for evaluating the object, based on the degree of attention and the degree of feeling calculated by the analysis unit. The determination unit calculates a viewing quality by multiplying the degree of attention and the degree of feeling, with respect to the object, calculated by the analysis unit. The determination unit calculates a viewing rate by multiplying an audience share and an access share, the audience share indicating a ratio of the number of terminals to be examined on which the object has been displayed, to the total number of terminals to be examined, the access share indicating a ratio of the number of at least one viewer whose gaze has accessed the object, to the total number of at least one viewer. The determination unit calculates the first of the visual object efficacy by multiplying the viewing quality and the viewing rate.

FIG.3

| | |
|---|---|
| INPUT ANALYSIS RESULTS (FOR EXAMPLE, DEGREE OF ATTENTION AND DEGREE OF FEELING) | S301 |
| CALCULATE ATTENTION RATE BASED ON DEGREE OF ATTENTION AND CALCULATE FEELING RATE BASED ON DEGREE OF FEELING | S302 |
| CALCULATE VIEWING QUALITY BASED ON ATTENTION RATE AND FEELING RATE | S303 |
| CALCULATE INSTANTANEOUS VISUAL ATTENTION RATE BASED ON VIEWING POINT AND ATTENTION RATE | S304 |
| CALCULATE INSTANTANEOUS VISUAL FEELING RATE BASED ON VIEWING POINT AND FEELING RATE | S305 |
| CALCULATE INSTANTANEOUS VISUAL OBJECT EFFICACY BASED ON VIEWING POINT AND VIEWING QUALITY | S306 |
| OUTPUT, FOR EXAMPLE, INSTANTANEOUS VISUAL OBJECT EFFICACY | S307 |

**Description**

Technical Field

The present invention relates to a visual object efficacy measuring device for measuring efficacy necessary for evaluating an object, such as content to be viewed by a human.

Background Art

Conventionally, it is known that pupil diameters of animals including humans expand when paying attention to an object with interest and concern. A technique for acquiring the degree of interest in and the degree of concern about an object by calculating a pupil diameter corresponding to the degree of attention has been proposed. Another technique has been proposed using an image recognition technique. The technique focuses on a feature, for example, an eye (around the eye, the center portion between the upper and lower sides of the eye, and a corner of the eye), an eyebrow (the inside, the center, and the outside), or a lip (the center between the upper and lower sides of the lip, and an end of the lip) of a human, and calculates a movement amount of the feature from a normal state. Based on a relative proportion of the movement amount of the feature, the degree of feeling indicating a type and an amount of feeling is acquired.

These techniques are used for measuring a physiological response of a viewer with respect to an object, such as content. For example, a device for determining emotion-and-feeling of a viewer, has been proposed (refer to Patent Literature 1). Using the viewer's feeling determination device can determines whether a viewer has viewed an object while paying attention, while being excited, or while being bored.

The viewer's feeling determination device calculates a pupil diameter from an eyeball image of the viewer and acquires the degree of attention based on the pupil diameter. In addition, the viewer's feeling determination device calculates a movement of a feature of a face from a face image of the viewer and a movement amount. The viewer's feeling determination device acquires the degree of feeling based on the movement of the face, and determines an emotion-and-feeling with respect to the object based on a movement of a visual point, the degree of attention, and the degree of feeling.

An emotion-and-feeling (EF) is a combination of an emotion (E) indicated with the degree of attention and a feeling (F) indicated with the degree of feeling. The emotion is a physiological response that involuntarily responds to an object. For example, in a case where a viewer has interest in and concern about the object, the viewer involuntarily pays attention to the object. As an amount of the physiological response, the degree of attention is acquired. The feeling includes a feeling that feels through the mind with respect to the object. For example, the feeling includes six basic feelings (pleasure, sadness, anger, surprise, disgust, and fear). As an amount of each of these feelings, the degree of feeling is acquired.

Citation List

Patent Literature

Patent Literature 1: WO 2011/042989

Summary of Invention

Technical Problem

The viewer's feeling determination device described in Patent Literature 1 above, converts the degree of attention into a numerical form for an emotion with respect to the object, and converts the degree of feeling into a numerical form for a feeling with respect to the object. The viewer's feeling determination device determines the emotion-and-feeling with respect to the object based on the degree of attention and the degree of feeling. Accordingly, a physiological response of the viewer to the object can be measured at a predetermined point in time.

However, the viewer's feeling determination device does not convert the emotion-and-feeling to be determined into a numerical form. Thus, there is a problem that the viewer's feeling determination device cannot sometimes measure a physiological response including a chronological element, correctly. Accordingly, upon determination of the emotion-and-feeling with respect to the object, the object is required to be further correctly evaluated.

Meanwhile, for example, in order to evaluate a program and a CM (commercial or advertisement) broadcast on television, a method is known based on a ratio of the number of television receivers on which a special TV program has been displayed to the number of television receivers that are terminals to be examined (activation rate), namely, an audience share. The method determines that evaluation is high in a case where the audience share is high, and that the evaluation is low in a case where the audience share is low. However, in the evaluation based on the audience share, even when the audience share is high, it cannot be determined whether the TV program and the CM have been just displayed on the television receiver while a viewer has been doing something else and a gaze of the viewer has been out of a television, whether the viewer has paid attention and viewed the TV program and the CM, whether the viewer has been bored and has viewed, or whether the viewer has been excited and has viewed. In a case where the viewer has paid attention and viewed the TV program and the CM, or in a case where the viewer has been excited and viewed, the evaluation should be high. In a case where the gaze of the viewer has been out of the television, or in a case where the viewer has been bored and viewed, the evaluation should be low. As described above, only the conventional evaluation using the audience share cannot correctly evaluate the television program and the CM themselves.

In order to evaluate the above television program and CM, the above method using the audience share and the method for determining an emotion-and-feeling by the viewer's feeling determination device described in Patent Literature 1 above, are preferably combined. However, a specific proposal has not been given yet.

Therefore, the present invention has been made in order to solve the problems. An object of the present invention is to provide a visual object efficacy measuring device capable of further correctly evaluating an object, such as content to be viewed by a human. Solution to Problem

A visual object efficacy measuring device according to the present invention includes: an analysis unit configured to acquire a pupil diameter from an eyeball image of at least one viewer viewing at least one object displayed on at least one terminal to be examined, calculate the degree of attention with respect to the at least one object based on the pupil diameter of the at least one viewer, acquire a predetermined part of a face of the at least one viewer from a face image of the at least one viewer, and calculate the degree of feeling with respect to the at least one object based on a movement of the predetermined part of the face of the at least one viewer; and a determination unit configured to calculate visual object efficacy as an evaluation value for evaluating the at least one object, based on the degree of attention and the degree of feeling calculated by the analysis unit. The determination unit calculates a viewing quality by multiplying the degree of attention and the degree of feeling calculated by the analysis unit, calculates a viewing rate by multiplying an audience share and an access share, the audience share indicating a ratio of the number of the at least one terminal to be examined on which the at least one object has been displayed to the total number of the at least one terminal to be examined, the access share indicating a ratio of the number of the at least one viewer whose gaze has accessed the at least one object to the total number of the at least one viewer, multiplies the viewing quality and the viewing rate, and defines a result acquired by the multiplication as the first of the visual object efficacy.

According to the visual object efficacy measuring device according to the present invention, the analysis unit further acquires visual-point coordinates indicating a position of the visual point of the at least one viewer, from the eyeball image of the at least one viewer, and generates access information indicating whether the gaze of the at least one viewer has accessed the at least one object, based on the visual-point coordinates. The determination unit further calculates, as visual-point-in-object time, time during which the gaze of the at least one viewer has accessed the at least one object, based on the access information generated by the analysis unit, calculates an access time rate, based on predetermined examination time and the visual-point-in-object time, calculates a viewing time rate by multiplying the audience share and the access time rate, and defines the viewing time rate as the second of the visual object efficacy.

According to the visual object efficacy measuring device of the present invention, the analysis unit further acquires visual-point coordinates indicating a position of the visual point of the at least one viewer, from the eyeball image of the at least one viewer, generates access information indicating whether the gaze of the at least one viewer has accessed the at least one object, based on the visual-point coordinates, and calculates, as an access viewer number, the number of the at least one viewer that has accessed the at least one object, based on the eyeball image or the face image. The determination unit further calculates as visual-point-in-object time, time during which the gaze of the at least one viewer has accessed the at least one object, based on the access information generated by the analysis unit, calculates an access time rate, based on predetermined examination time and the visual-point-in-object time, calculates an access time viewer number rate by multiplying the access time rate and the access viewer number calculated by the analysis unit, and defines the access time viewer number rate as the third of the visual object efficacy.

According to the visual object efficacy measuring device of the present invention, the determination unit calculates pieces of the visual object efficacy for a plurality of the objects included in the same category, and the pieces of the visual object efficacy rates in descending order.

According to the visual object efficacy measuring device of the present invention, the at least one object is defined as video content. The determination unit calculates the visual object efficacy of the video content, compares the visual object efficacy and a predetermined reference value. In a case where the visual object efficacy is lower than the reference value, the determination unit causes the at least one viewer to view other video content, calculates and compares the visual object efficacy of the other video content to the reference value, performs the calculation and comparison processing until the determination unit selects video content in which the visual object efficacy becomes the reference value or more, and causes the at least one viewer to view different video content during a period of time until the visual object efficacy becomes the reference value or more.

According to the visual object efficacy measuring device of the present invention, the at least one object is defined as video content. The determination unit further compares the visual object efficacy and a predetermined value, calculates, as the degree of excitement, a ratio of time during which the visual object efficacy has been larger than the predetermined value to predetermined evaluation object time during which the at least one viewer has accessed the at least one object. In a case where the degree of excitement is larger than a predetermined threshold value, the determination unit determines that the video content is content at which the at least one viewer becomes excited. In a case where the degree of excitement is the predetermined threshold value or less, the determination unit determines that the video content is content at which the at least one viewer does not become excited.

According to the visual object efficacy measuring device of the present invention, the at least one object is defined as a commodity. The determination unit calculates a positive viewing quality by multiplying the degree of attention and the degree of feeling of a feeling of pleasure, with respect to the at least one object, calculated by the analysis unit, multiplies the positive viewing quality and the viewing rate, and defines a result acquired by the multiplication as positive visual object efficacy. In a case where the positive visual object efficacy is larger than a predetermined threshold value, the determination unit determines that the commodity is goods in which the degree of possibility of purchase is high. In a case where the positive visual object efficacy is the predetermined threshold value or less, the determination unit determines that the commodity is goods in which the degree of possibility of purchase is low.

Advantageous Effects of Invention

As described above, according to the present invention, an object, such as content to be viewed by a human, can be further correctly evaluated.

Brief Description of Drawings

Fig. 1 is a schematic diagram of a configuration of a visual object efficacy measuring system including a visual object efficacy measuring device according to an embodiment of the present invention.
Fig. 2 is a block diagram of a configuration of the visual object efficacy measuring device according to the embodiment of the present invention.
Fig. 3 is a flow chart of processes of a determination unit included in the visual object efficacy measuring device according to the embodiment of the present invention.
Fig. 4 is a graphical representation for describing the degree of excitement with respect to video content.
Fig. 5 is a graphical representation for describing the degree of possibility of purchase with respect to a commodity.
Fig. 6 is a schematic diagram of a configuration of a visual object efficacy measuring system including a visual object efficacy measuring device according to a second embodiment of the present invention.
Fig. 7 is a view for describing external appearances of an image display, a face capturing device, an eyeball measuring device, a brightness measuring device, and the visual object efficacy measuring device.
Fig. 8 is a block diagram of a configuration of the visual object efficacy measuring device according to the second embodiment of the present invention.
Fig. 9 is a flow chart of first processing of a determination unit included in the visual object efficacy measuring device according to the second embodiment of the present invention.
Fig. 10 is a flow chart of second processing of the determination unit included in the visual object efficacy measuring device according to the second embodiment of the present invention.
Fig. 11 is a flow chart of processing of the determination unit in an example of rating an object.
Fig. 12 is a flow chart of processing of the determination unit in an example of selecting video content.
Fig. 13 is a flow chart of processing of the determination unit in an example of determining the degree of excitement with respect to the video content.
Fig. 14 is a flow chart of processing of the determination unit in an example of measuring the degree of possibility of purchase with respect to a commodity.
Fig. 15 is a diagram for describing a concept of a determination value calculated by the determination unit (evaluation data).
Fig. 16 is a diagram of connections between pieces of data and expressions upon calculation of instantaneous evaluation data of an individual viewer with respect to an object in Fig. 15(1).
Fig. 17 is a diagram of connections between pieces of data and expressions upon calculation of instantaneous evaluation data of all viewers with respect to the same object in Fig. 15(3).
Fig. 18 is a diagram for describing relationships between each of pieces of data in the instantaneous evaluation data of all the viewers with respect to the same object in Fig. 15(3) in a case where the object is shared.

Fig. 19 is a diagram for describing relationships between each of pieces of data in a case where an object is a TV program.

Fig. 20 is a diagram for describing relationships between each of pieces of data in a case where the object is, for example, a CM displayed on, for example, a personal computer.

Fig. 21 is a diagram for describing relationships between each of pieces of data in a case where the object is on a WEB screen.

Fig. 22 is a diagram for describing relationships between each of pieces of data in a case where the object is a piece of printed matter (electronic book in the present example).

Fig. 23 is a diagram for describing relationships between each of pieces of data in a case where the object is a display board, such as a poster or a sign board.

Fig. 24 is a diagram for describing relationships between each of pieces of data in a case where the object is a commodity.

Description of Embodiments

Embodiments of the present invention will be described in detail below using the drawings.

(Visual object efficacy measuring system)

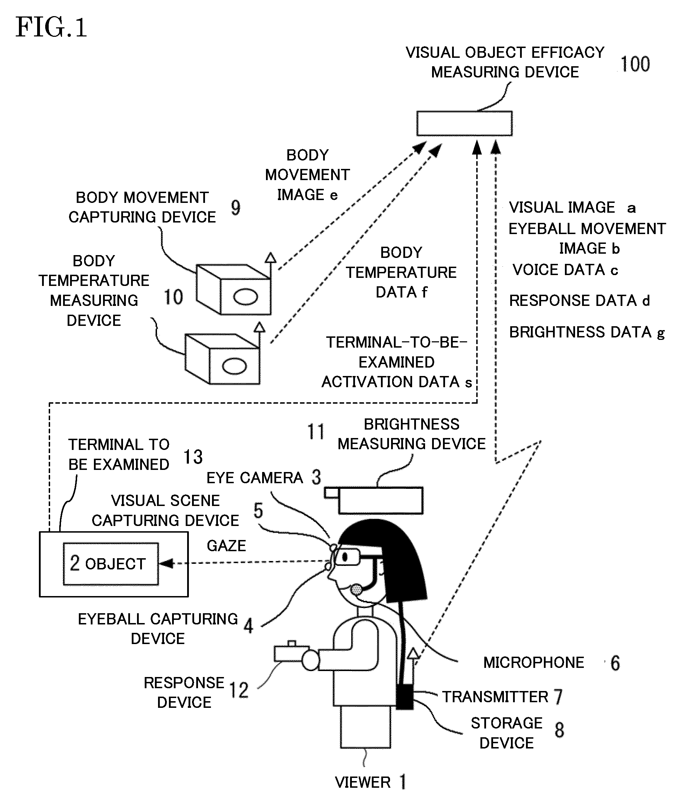

First, a configuration and each configuration unit of a visual object efficacy measuring system including a visual object efficacy measuring device according to an embodiment of the present invention, will be described. Fig. 1 is a schematic diagram of the configuration of the visual object efficacy measuring system including the visual object efficacy measuring device according to the embodiment of the present invention. The visual object efficacy measuring system includes an eye camera 3, a transmitter 7, a storage device 8, a body movement capturing device 9, a body temperature measuring device 10, a brightness measuring device 11, a response device 12, a terminal to be examined 13, and the visual object efficacy measuring device 100. The eye camera 3 includes an eyeball capturing device 4, a visual scene capturing device 5, and a microphone 6.

A viewer 1 wears the eye camera 3 on the head, and a gaze of the viewer 1 faces a visual scene including the terminal to be examined 13 on which an object to be viewed 2 is displayed. A device corresponding to the eye camera 3 may be fixed near the terminal to be examined 13. Here, the visual scene may include anything as long as it can be viewed by the viewer 1 as a scene. For example, the visual scene includes a commodity, a store, an open space, a human, and a streetscape. In addition, the visual scene includes content of image data, web page data, computer game data, and data output from a computer program that are displayed on a display of, for example, a computer, a mobile phone, or a television. As data displayed on the terminal to be examined 13, response evocating information for causing the viewer 1 to respond, such as a character string, a figure, a symbol, a picture, a photograph, or a moving image, is displayed. The data includes response object information, such as the character string, the figure, the symbol, the picture, the photograph, or the moving image, to be an object to which the viewer 1 responds.

Examples of the object 2 include a display image (TV program, and video content, still image content, and a WEB screen displayed on, for example, a computer or a mobile terminal), a piece of printed matter (electronic book in the present example), a display board such as a poster or a sign board, and a commodity.

As described above, the eye camera 3 includes the eyeball capturing device 4, a visual scene capturing device 5, and the microphone 6. In Fig. 1, the eye camera 3 is coupled to the transmitter 7 and the storage device 8. The viewer 1 also wears the transmitter 7 and the storage device 8. In a case where the visual scene includes the terminal to be examined 13, such as a computer, a mobile phone, or a television receiver, the eyeball capturing device 4 and the microphone 6 may be disposed on the side of the terminal to be examined 13 without wearing the eye camera 3 on the head.

The eyeball capturing device 4 captures an eye ball of the viewer 1. An eyeball movement image b captured by the eyeball capturing device 4 is stored in the storage device 8. Note that, the eyeball movement image b may be stored as a moving image or as still images that have been periodically captured.

The visual scene capturing device 5 captures the visual scene including the object 2 faced by the gaze of the viewer 1. A visual image a captured by the visual scene capturing device 5 is stored in the storage device 8. Note that, the visual image a may be stored as a moving image or as still images that have been periodically captured.

The microphone 6 captures a voice of the viewer 1 and a sound around the viewer 1. Voice data c captured by the microphone 6 is stored in the storage device 8.

The body movement capturing device 9 captures a movement of each of parts of the body of the viewer 1 including the face. The body movement capturing device 9 transmits a body movement image e that has been captured, to the visual object efficacy measuring device 100 by wireless communication or wired communication. In this case, the body movement capturing device 9 may transmit the body movement image e to the visual object efficacy measuring device 100 at predetermined time intervals. The body movement capturing device 9 may transmit the body movement image e to the visual object efficacy measuring device 100 in accordance with an instruction from the visual object efficacy measuring device 100. The body movement image e captured by the body movement capturing device 9 includes a face image of the viewer 1. A movement and an expression of the face are analyzed based on the body movement image e. Note that a face capturing device for capturing only the face of the viewer 1 may be prepared anew. For example, the face capturing device can be fit to a head end of a peak of a cap-type eye camera 3 so that the face can be captured.

The body temperature measuring device 10 measures a temperature of the body of the viewers 1. The body temperature measuring device 10 transmits body temperature data f to the visual object efficacy measuring device 100 by wired communication or wireless communication. In this case, the body temperature measuring device 10 may transmit the body temperature data f to the visual object efficacy measuring device 100 at predetermined time intervals. The body temperature measuring device 10 may transmit the body temperature data f to the visual object efficacy measuring device 100 in accordance with an instruction from the visual object efficacy measuring device 100. In Fig. 1, the body temperature measuring device 10 remotely measures the temperature of the body of the viewer 1. A sensor worn on the viewer 1 may directly measure the temperature. In this case, the body temperature data f that has been measured is stored in the storage device 8. The transmitter 7 transmits the body temperature data f to the visual object efficacy measuring device 100.

Here, a capturing frequency of an image frame in each of the eyeball capturing device 4, the visual scene capturing device 5, and the body movement capturing device 9 is suitably a high speed of 60 Hz or more. The capturing frequency of an image frame in each of the eyeball capturing device 4, the visual scene capturing device 5, and the body movement capturing device 9 is preferably the same.

The brightness measuring device 11 has a function for measuring brightness data g of the visual scene. For example, the brightness measuring device 11 measures brightness of a display of the terminal to be examined 13. The brightness data g is stored in the storage device 8.

The response device 12 acquires a response signal based on a movement of the viewer 1. Examples of the movement of the viewer 1 include a button pressing operation, a keyboard operation, a mouse operation, a touch panel operation, a remote control operation, an operation of a controller included in a game machine, an operation of a machine, and other body actions, such as raising of a hand or vocalization. Response data d acquired by the response device 12 is stored in the storage device 8.

The storage device 8 stores, on a time series basis, the visual image a from the visual scene capturing device 5, the eyeball movement image b from the eyeball capturing device 4, the voice data c from the microphone 6, the response data d from the response device 12, and the brightness data g from the brightness measuring device 11 as pieces of data synchronized with the body movement image e captured by the body movement capturing device 9 and the body temperature data f measured by the body temperature measuring device 10.

The transmitter 7 transmits the visual image a, the eyeball movement image b, the voice data c, the response data d, and the brightness data g that have been stored in the storage device 8, to the visual object efficacy measuring device 100 by wireless communication or wired communication. In this case, the transmitter 7 may transmit each of the pieces of data to the visual object efficacy measuring device 100 at predetermined time intervals. The transmitter 7 may transmit each of the pieces of data to the visual object efficacy measuring device 100 in accordance with an instruction from the visual object efficacy measuring device 100.

In Fig. 1, the eye camera 3 and the response device 12 have been coupled to the storage device 8. Each of pieces of data is transmitted to the storage device 8 by wired communication. Each of the pieces of data may be transmitted to the storage device 8 by wireless communication. In a case where wireless communication is used, each of the pieces of data of the eyeball capturing device 4, the visual scene capturing device 5, the microphone 6, the brightness measuring device 11, and the response device 12, may be directly transmitted to the visual object efficacy measuring device 100.

The terminal to be examined 13 displays the object 2 on the display of the terminal to be examined 13 (activate the object 2), generates terminal-to-be-examined activation data s (1 or 0) indicating whether the object 2 has been displayed (activated), and transmits the terminal-to-be-examined activation data s to the visual object efficacy measuring device 100 by wired communication or wireless communication. For the terminal-to-be-examined activation data s, in a case where an image previously set has been displayed on the terminal to be examined 13 (in a case where the terminal to be examined 13 is a television receiver, and a special TV program) as the object 2, data (1) indicating activation is set. In a case where another image has been displayed on the terminal to be examined 13, the image is determined not to be the object 2 and data (0) indicating no activation is set. That is, even when any image has been displayed, the terminal to be examined 13 generates the terminal-to-be-examined activation data s(0) indicating no activation in a case where the image previously set has not been displayed.

Note that, in a case where an object 2 that is a special image is displayed to a viewer 1 who is a subject, so as to evaluate the object 2, for example, in a market survey, a terminal to be examined 13 continuously displays the object 2 that is the special image. In this case, the terminal to be examined 13 continuously generates the terminal-to-be-examined activation data s(1) indicating that the object 2 has been activated.

(Visual object efficacy measuring device 100)

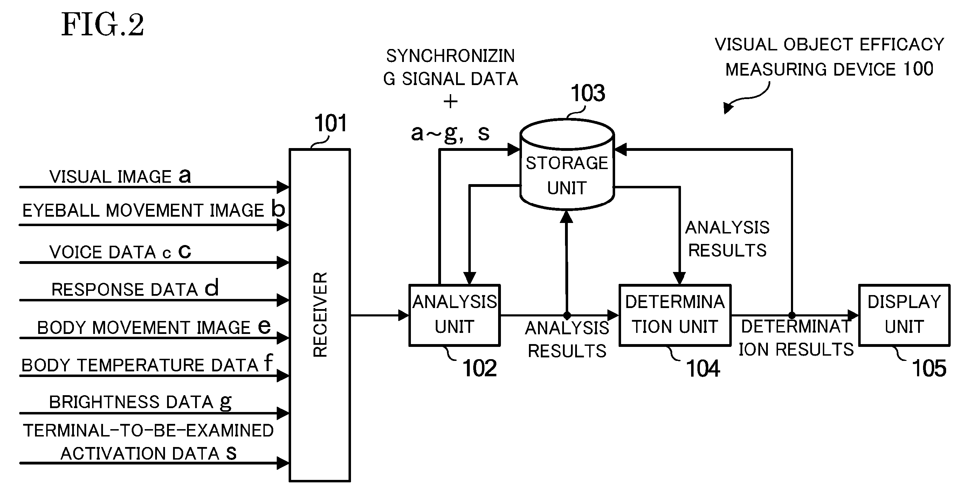

Next, a configuration and each configuration unit of the visual object efficacy measuring device 100 illustrated in Fig. 1, will be described. Fig. 2 is a block diagram of the configuration of the visual object efficacy measuring device 100 according to the embodiment of the present invention. The visual object efficacy measuring device 100 includes a receiver 101, an analysis unit 102, a storage unit 103, a determination unit 104, and a display unit 105.

Note that the visual object efficacy measuring system illustrated in Fig. 1 may include a communication device instead of the visual object efficacy measuring device 100. The communication device may be made to be coupled to the visual object efficacy measuring device 100 that functions as a server, through a communication network, such as the Internet. In this case, the visual object efficacy measuring device 100 functioning as a server is coupled to a communication device of each of a plurality of visual object efficacy measuring systems. The visual object efficacy measuring device 100 collects the pieces of data a to g, and s each of the plurality of visual object efficacy measuring systems. The visual object efficacy measuring device 100 to be described below (in particular, the analysis unit 102 and the determination unit 104) includes a function as a server in addition to a function as a device in the visual object efficacy measuring system illustrated in Fig. 1. The same is true of a second embodiment to be described later.

The visual object efficacy measuring device 100 illustrated in Fig. 1 may be made to be coupled to a data-aggregate-and-statistics center through a network, such as the internet, as in the second embodiment to be described later. In this case, the data-aggregate-and-statistics center receives the pieces of data a to g, and s, analysis results and determination results to be described later, from the communication unit (not illustrated) included in the visual object efficacy measuring device 100 in each of the plurality of visual object efficacy measuring system. The data-aggregate-and-statistics center performs pieces of processing similar to those of the receiver 101, the analysis unit 102 and the determination unit 104 to be described later.

(Receiver)

The receiver 101 receives the visual image a, the eyeball movement image b, the voice data c, the response data d, and the brightness data g from the transmitter 7. The receiver 101 receives the body movement image e from the body movement capturing device 9, the body temperature data f from the body temperature measuring device 10, and the terminal-to-be-examined activation data s from the terminal to be examined 13. The receiver 101 synchronizes all the pieces of data that have been received, and outputs each of the pieces of data to the analysis unit 102 at predetermined time intervals or in accordance with an instruction from the analysis unit 102.

In order to synchronize the pieces of data that have been received, each of the pieces of data is made so as to include time of a clock as a synchronizing signal. Note that the visual object efficacy measuring device 100 may generate the synchronizing signal based on the time of a clock and transmits the synchronizing signal that has been generated, to the transmitter 7, the body movement capturing device 9, the body temperature measuring device 10, and the terminal to be examined 13. The transmitter 7 may transmit the visual image a, the eyeball movement image b, the voice data c, the response data d, and the brightness data g at timing of the synchronizing signal that has been received, together with the synchronizing signal to the visual object efficacy measuring device 100. In addition, the body movement capturing device 9 may transmit the body movement image e at the timing of the synchronizing signal that has been received, together with the synchronizing signal to the visual object efficacy measuring device 100. The body temperature measuring device 10 may transmit the body temperature data f at the timing of the synchronizing signal that has been received, together with the synchronizing signal to the visual object efficacy measuring device 100. The terminal to be examined 13 may transmit the terminal-to-be-examined activation data s at the timing of the synchronizing signal that has been received, together with the synchronizing signal to the visual object efficacy measuring device 100. Accordingly, all the pieces of data are synchronized so that the processing can be performed.

Note that in a case where the visual object efficacy measuring device 100 functions as a server and is coupled to a communication device of each of the plurality of visual object efficacy measuring systems, the communication device of each of the plurality of visual object efficacy measuring systems transmits the pieces of data a to g, and s to the visual object efficacy measuring device 100. The receiver 101 of the visual object efficacy measuring device 100 receives the pieces of data a to g, and s from each of the communication devices.

For example, in a case where there is a plurality of viewers 1 with respect to one terminal to be examined 13, the receiver 101 receives pieces of data a to g from each of the viewers 1. For example, in a case where there is a plurality of terminals to be examined 13, the receiver 101 receives a piece of data s from each of the plurality of terminals to be examined 13.

(Analysis unit)

The receiver 101 inputs the visual image a, the eyeball movement image b, the voice data c, the response data d, the body movement image e, the body temperature data f, the brightness data g, and the terminal-to-be-examined activation data s to the analysis unit 102, at predetermined time intervals or by giving an instruction to the receiver 101.

The analysis unit 102 adds the synchronizing signal to the pieces of data a to g, and s. The analysis unit 102 stores the pieces of data a to g, and s with synchronizing signals in the storage unit 103. As necessary, the analysis unit 102 reads the pieces of data a to g, and s that have been stored in the storage unit 103. The analysis unit 102 uses the data a to g, and s that have been read or the data a to g, and s that have been input, for analysis, and generates analysis results. The analysis unit 102 stores the analysis results that have been generated, in the storage unit 103, and outputs the analysis results to the determination unit 104.

More specifically, the analysis unit 102 generates, as analysis results, for example, the degree of attention with respect to an object 2, degree-of-attention changing speed, degree-of-attention strengthening acceleration, visual-point tracking time, visual-point approaching speed, visual-point approaching acceleration, visual-point averting speed, visual-point averting acceleration, and the degree of feeling. The analysis unit 102 calculates, as analysis results, an access point, a viewing point, instantaneous audience share, and instantaneous access rate. The access point is visual-point access/no visual-point access information indicating whether a viewer 1 has accessed a region of the object 2 (region previously set in a terminal to be examined 13). The viewing point indicates whether the viewer 1 has practically accessed the object 2 displayed on the terminal to be examined 13. The instantaneous audience share is based on the number of terminals to be examined 13. The instantaneous access share is based on the number of viewers 1. Detailed descriptions of the access point, the viewing point, the instantaneous audience share, and the instantaneous access share, will be given later. Displaying the object 2 on the terminal to be examined 13 means that the object 2 has been activated on the terminal to be examined 13.

For example, the analysis unit 102 calculates a position and a movement of a visual point (for example, a moved distance) from the visual image a and the eyeball movement image b, calculates a movement of the head (for example, a moved distance) from the body movement image e, and calculates a movement of the object 2 from the visual image a. The analysis unit 102 calculates a pupil diameter from the eyeball movement image b, calculates a pupil diameter that eliminates a pupil diameter changing part corresponding to brightness of the object 2 from the brightness data g, and calculates the degree of attention based on the pupil diameter. The analysis unit 102 grasps each of parts of features of the face of the viewer 1 (for example, an eye, a lip, and an eyebrow) by an image analysis technique using viewer's face image data included in the body movement image e. The analysis unit 102 measures a variation of each of the parts. The analysis unit 102 previously calculates a value of the movement of each of the parts of the face when the viewer 1 is in a normal state, and a maximum value of the movement of each of the parts of the face when the viewer 1 expresses a special feeling (reference maximum value). In addition, the analysis unit 102 previously calculates a ratio between the value and the maximum value (reference ratio). The analysis unit 102 calculates a ratio between a value of a movement of each of the parts of the face that has been calculated in actual measurement and the reference maximum value. The analysis unit 102 calculates the degree of feeling indicating an expression of the face and a magnitude thereof using the ratio that has been calculated and the reference ratio.

The degree of feeling is calculated for each of feelings of six basic feelings (pleasure, sadness, anger, surprise, disgust, and fear). At the same timing, any one feeling of the six basic feelings that has been expressed is calculated as the degree of feeling. A feeling of pleasure may be defined as a positive feeling. Four feelings including sadness, anger, disgust, and fear, each may be defined as a negative feeling. Each of the positive feeling and the negative feelings may be calculated as the degree of feeling.

Note that processing for generating analysis results, such as the degree of attention and the degree of feeling by the analysis unit 102, has been known. For the detailed descriptions of the processing, refer to Patent Literature 1 above.

Next, the access point, the viewing point, the instantaneous audience share, and the instantaneous access share calculated by the analysis unit 102, will be described. As described above, the analysis unit 102 calculates the position of the visual point of the viewer 1 from the visual image a and the eyeball movement image b of the viewer 1, acquires visual-point coordinates indicating the position of the visual point, and calculates the access point (1 or 0) that is the visual-point access/no visual-point access information indicating whether the gaze of the viewer 1 has accessed the region of the object 2 (region previously set) from the visual-point coordinates. Even when there is no object 2, in a case where the gaze of the viewer 1 has accessed the region that has previously been set (region in which there is the object 2), the access point is set to be 1.

Here, in a case where the terminal to be examined 13 has displayed (activated) the object 2 (terminal-to-be-examined activation data s (activation point) = 1) and the gaze of the viewer 1 has accessed the object 2 (access point = 1), the viewer 1 has actually viewed the object 2. In this case, the viewing point = 1 is defined. For example, in a case where the terminal to be examined 13 is a television receiver, a special TV program has been displayed as the object 2. A state where the viewing point = 1 is satisfied, indicates that the viewer 1 has actually viewed the TV program. In a case where the special TV program has not been displayed as the object 2 (activation point = 0), even when the viewer 1 has viewed the television receiver (access point = 1), the viewer 1 has not viewed the special TV program (viewing point = 0). Even in a case where the special TV program has been displayed as the object 2 (activation point = 1), when the viewer 1 has not viewed the TV program (access point = 0), the viewer 1 has not viewed the TV program (viewing point = 0).

That is, the analysis unit 102 calculates the viewing point indicating whether the viewer 1 has actually viewed the object 2, from the activation point of the terminal-to-be-examined activation data s and the access point, by the following expression.

[Expression 1]

$$\text{Viewing point (1 or 0)} = \text{Activation point (1 or 0)} \times \text{Access point (1 or 0)} \dots (1)$$

For example, in a case where the visual object efficacy measuring device 100 functions as a server coupled to a communication device of each of a plurality of visual object efficacy measuring systems through a network, the analysis unit 102 inputs pieces of data a to g, and s of an individual viewer 1 of each of the plurality of the visual object efficacy measuring systems, from the receiver 101. As described above, the analysis unit 102 calculates a viewing point of each of all the viewers 1. The analysis unit 102 performs a statistical calculation of instantaneous data with respect to all the viewers 1 as in the following.

The analysis unit 102 counts the number of activation points each that have indicated 1 and that are the terminal-to-be-examined activation data s (the number of terminals to be examined 13 that have displayed the object 2). The analysis unit 102 calculates a count value as an instantaneous visual object activation number. The analysis unit 102 calculates the instantaneous audience share based on the instantaneous visual object activation number and the number of terminals to be examined 13 that have been previously set (a total number of terminals to be examined). The instantaneous audience share indicates a ratio of the instantaneous visual object activation number that is the number of the terminals to be examined 13 each having displayed the object 2 (the object 2 has been activated) to the total number of terminals to be examined that have been previously set.

[Expression 2]

$$\text{Instantaneous audience share} = \text{Instantaneous visual object activation number/Total number of terminals to be examined} \dots (2)$$

In a case where the terminal to be examined 13 is a television receiver and the object 2 is a special TV program, the terminal-to-be-examined activation data s is set to be 1 when the special TV program has been displayed (a special channel has been selected). The analysis unit 102 counts the number of activation points each that have indicated 1 and that are the terminal-to-be-examined activation data s (the number of television receivers in which the special channel has been selected). The analysis unit 102 calculates a count value as the number of television receivers in which the special channel has been selected (special channel selection TV number). The analysis unit 102 calculates a TV program instantaneous audience share based on the special channel selection TV number (the number of television receivers that have displayed the special TV program) and the number of television receivers that have been previously set (a total TV-to-be-examined number). The TV program instantaneous audience share indicates a ratio of the special channel selection TV number that is the number of television receivers in which the special channel has been selected, to the total TV-to-be-examined number previously set.

[Expression 3]

$$\text{TV program instantaneous audience share} = \text{Special channel selection TV number/Total TV-to-be-examined number} \dots (3)$$

The analysis unit 102 counts the number of access points each that have indicated 1 (the number of viewers 1 whose gazes have accessed the region of the object 2), and calculates a count value as an instantaneous access viewer number. The analysis unit 102 calculates the number of access points each that have indicated 1 or 0, as the number of viewers 1 (total viewer number). In this case, the analysis unit 102 may calculate, for example, the number of viewing points, the number of eyeball movement images b as the number of viewers 1 (total viewer number) instead of the number of access points. A number that has been previously set may be used for the number of viewers 1 (total viewer number). The analysis unit 102 calculates the instantaneous access share based on the instantaneous access viewer number and the total viewer number by the following expression. The instantaneous access share indicates a ratio of the instantaneous access viewer number that is the number of viewers 1 whose gazes have accessed the region of the object 2, to the total viewer number that has been previously set.

[Expression 4]

$$\text{Instantaneous access share} = \text{Instantaneous access viewer number}/\text{Total viewer number} \dots (4)$$

(Storage unit)

The storage unit 103 stores the visual image a, the eyeball movement image b, the voice data c, the response data d, the body movement image e, the body temperature data f, the brightness data g, and the terminal-to-be-examined activation data s, and the synchronizing signal that have been output by the analysis unit 102. The storage unit 103 storages the analysis results that have been output by the analysis unit 102. Furthermore, the storage unit 103 stores determination results output by the determination unit 104 to be described later.

Note that the analysis unit 102 has been made to calculate the instantaneous audience share by Expression (2) above, the TV program instantaneous audience share by Expression (3) above, and the instantaneous access share by Expression (4) above. These pieces of data may be input from an external device or input in accordance with an operation of an operator so as to be stored in the storage unit 103. Furthermore, a measuring unit, not illustrated, included in the visual object efficacy measuring device 100 may measure these pieces of data by a known method. The analysis unit 102 may input these pieces of data from the measuring unit. Then, the analysis unit 102 may store these pieces of data in the storage unit 103.

(Determination unit)

The determination unit 104 inputs, for example, the degree of attention and the degree of feeling that are analysis results, from the analysis unit 102. The determination unit 104 calculates the degree of emotion-and-feeling based on the degree of attention and the degree of feeling. The degree of emotion-and-feeling is a value acquired by converting an emotion-and-feeling including an emotion and a feeling combined into a numerical form. The degree of emotion-and-feeling corresponds to a viewing quality to be described later.

The determination unit 104 calculates, for example, visual object efficacy as a determination value for evaluating the object 2, based on the degree of emotion-and-feeling. The determination unit 104 outputs these determination results to the display unit 105. Examples of the determination value include the viewing quality, a viewing rate, the visual object efficacy in an instant in which a gaze has accessed in order for the viewer 1 to view the object 2, and during predetermined examination time (examination period). That is, the determination value includes evaluation data at an instantaneous point in time for evaluating the object 2 and evaluation data acquired by statistically calculating the evaluation data at the instantaneous point in time, during the predetermined examination time.

Note that, the determination unit 104 may read, for example, the degree of attention and the degree of feeling of analysis results, from the storage unit 103.

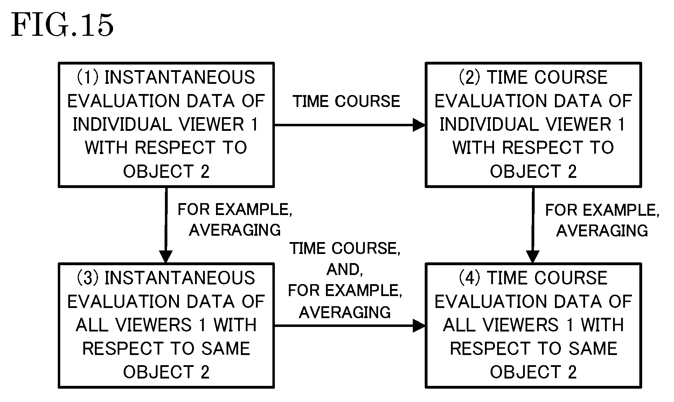

Fig. 15 is a diagram for describing a concept of the determination value (evaluation data) calculated by the determination unit 104. Fig. 15 illustrates a relationship in a case where the determination value for evaluating the object 2 is divided into instantaneous evaluation data and time course evaluation data. The determination value calculated by the determination unit 104 is divided into instantaneous evaluation data (1) of an individual viewer 1 with respect to an object 2, time course evaluation data (2) of the individual viewer 1 with respect to the object 2, instantaneous evaluation data (3) of all viewers 1 with respect to the same object 2, and time course evaluation data (4) of all the viewers 1 with respect to the same object 2.

In Fig. 15(1), the instantaneous evaluation data of the individual viewer 1 with respect to the object 2, indicates an instantaneous determination value for the individual viewers 1. In Fig. 15(2), the time course evaluation data of the individual viewer 1 with respect to the object 2, indicates a time series determination value that includes the instantaneous evaluation data in Fig. 15(1) varying as time passes. In Fig. 15(3), the instantaneous evaluation data of all the viewers 1 with respect to the same object 2 indicates a determination value when pieces of the instantaneous evaluation data in Fig. 15(1) of all the viewers 1 are added together and then an average value or a maximum value is calculated. In Fig. 15(4), the time course evaluation data of all the viewers 1 with respect to the same object 2, indicates a determination value when pieces of the instantaneous evaluation data in Fig. 15(3) changed during the predetermined examination time, as time passes, are added together, and then average value or a maximum value is calculated. In Fig. 15(4), the time course evaluation data of all the viewers 1 with respect to the same object 2 indicates a determination value when pieces of the time course evaluation data in Fig. 15(2) are added together for all the viewers 1 and then an average value or a maximum value is calculated.

For example, in a case where the object 2 is a TV program and the terminal to be examined 13 is a television receiver, in a state where one viewer 1 has viewed the TV program, the determination unit 104 inputs analysis results generated by the analysis unit 102 and calculates the instantaneous evaluation data of the individual viewer 1 with respect to the object 2 in Fig. 15(1). In a state where one viewer 1 has viewed the TV program from the beginning to the end of the predetermined examination time, the determination unit 104 inputs the analysis results generated by the analysis unit 102 and calculates the time course evaluation data of the individual viewer 1 with respect to the object 2 in Fig. 15(2). In Fig. 15(2), the time course evaluation data of the individual viewer 1 with respect to the object 2 is evaluation data of the individual viewer 1 with respect to the TV program to be examined during the predetermined time period. Therefore, the time course evaluation data can be handled as evaluation data of the individual viewer 1 with respect to entire content of the TV program. In Fig. 15(1), the instantaneous evaluation data of the individual viewer 1 with respect to the object 2 is data of a certain instant segmented with respect to the time course evaluation data of the individual viewer 1 with respect to the object 2 in Fig. 15(2).

For all television receivers and all the viewers 1, evaluation data calculated based on pieces of instantaneous data a to g, and s of the same TV program is the instantaneous evaluation data of all the viewers 1 with respect to the same object 2 in Fig. 15(3).

As data of all viewers 1 who have viewed the same TV program, pieces of the time course evaluation data in Fig. 15(2) are superimposed so as to calculate the time course evaluation data of all the viewers 1 with respect to the same objects 2 in Fig. 15(4). The time course evaluation data can be handled as evaluation data of all the viewers 1 with respect to entire content of the TV program. The instantaneous evaluation data in Fig. 15(3) is expressed in a time course form from the beginning to the end of the predetermined examination time of the TV program as the data of all the viewers 1 who have viewed the same TV program. Thus, the time course evaluation data of all the viewers 1 is calculated with respect to the same object 2 in Fig. 15(4). The time course evaluation data (time course evaluation data in Fig. 15(4) calculated based on the instantaneous evaluation data in Fig. 15(3)) is the same as the time course evaluation data in Fig. 15(4) calculated based on the time course evaluation data in Fig. 15(2).

Note that, examples of a method for calculating the time course evaluation data of all the viewers 1 with respect to the same object 2 in Fig. 15(4) by the determination unit 104 includes processing for superimposing the pieces of time course evaluation data in Fig. 15(2) as data of all the viewers 1, and processing for expressing the instantaneous evaluation data in Fig. 15(3) in the time course form. Both of the pieces of processing are different from each other. The determination unit 104 uses any of the pieces of calculation processing, depending on a purpose of examination.

(Calculation processing for instantaneous evaluation data of individual viewer 1 with respect to object 2: Fig. 15(1))

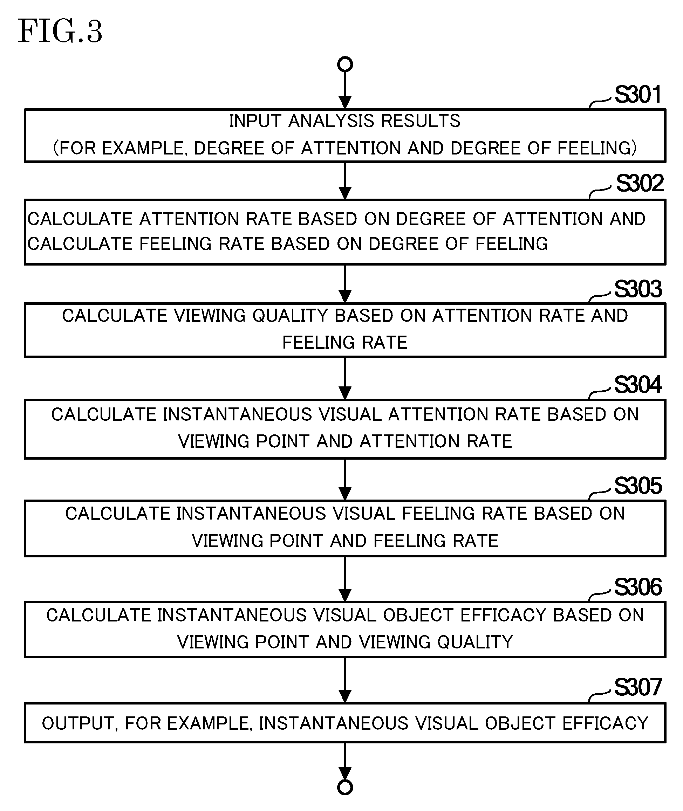

First, calculating processing for the instantaneous evaluation data of the individual viewer 1 with respect to the object 2 in Fig. 15(1) by the determination unit 104 will be described. Fig. 3 is a flow chart of the processing of the determination unit 104. Fig. 3 illustrates the processing for calculating the instantaneous evaluation data of the individual viewer 1 with respect to the object 2 in Fig. 15(1). First, the determination unit 104 inputs the analysis results (for example, the degree of attention and the degree of feeling) from the analysis unit 102 (Step S301), calculates an attention rate (instantaneous attention rate) from the degree of attention, and also calculates a feeling rate (instantaneous feeling rate) from the degree of feeling (Step S302).

[Expression 5]

$$\text{Attention rate (\%)} = \text{Degree of attention} \times 100/2 \ ... \ (5)$$

[Expression 6]

$$\text{Feeling rate (\%)} = \text{Degree of feeling} \times 100/5 \ ... \ (6)$$

Here, the degree of attention is a value of from -2 to 2. The degree of feeling is a value of from 0 to 5. In this case, the analysis unit 102 calculates the degree of attention in a range between -2 and 2 and calculates the degree of feeling in a range between 0 and 5. The determination unit 104 inputs the degree of attention having a value of the range and the degree of feeling having a value of the range, from the analysis unit 102. Accordingly, the attention rate calculated by the determination unit 104 has a value of from -100 to 100%. The feeling rate calculated by the determination unit 104 has a value of from 0 to 100%.

As described above, feeling includes the six basic feelings (pleasure, sadness, anger, surprise, disgust, and fear). A feeling of pleasure is defined as a positive feeling. Four feelings including sadness, anger, disgust, and fear, each are defined as a negative feeling. The feeling rate may be made to be calculated for each of the feelings. The same is true of data regarding a feeling, such as an instantaneous visual feeling rate to be described later.

The determination unit 104 calculates the viewing quality of the viewer 1 (instantaneous viewing quality) based on the attention rate and the feeling rate that have been calculated at Step S302, by the following expression (Step S303).

[Expression 7]

$$\text{Viewing quality} = \{k1 \times \text{Attention rate (\%)}\} \times \{k2 \times \text{Feeling rate (\%)}\}/100 \ldots (7)$$

Here, the viewing quality is the product of the attention rate and the feeling rate when the viewer 1 has viewed the object 2. The viewing quality is quality data of the object 2 that corresponds to the degree of emotion-and-feeling in which an emotion-and-feeling including an emotion and a feeling combined has been converted into a numerical form. k1 is a weighting coefficient of the attention rate, and the range satisfies $0 \le k1 \le 10$. k2 is a weighting coefficient of the feeling rate, and the range satisfies $0 \le k2 \le 10$. The weighting coefficients k1 and k2 are previously and arbitrarily set in accordance with a purpose of evaluation of the object 2 when the viewer 1 has viewed the object 2.

The determination unit 104 calculates an instantaneous visual attention rate of the viewer 1 based on the viewing point that has been input at Step S301 and the attention rate that has been calculated at Step S302, by the following expression (Step S304).

[Expression 8]

$$\text{Instantaneous visual attention rate} = \text{Viewing point (1 or 0)} \times \text{Attention rate} \ldots (8)$$

The instantaneous visual attention rate is a value indicating with what degree of attention rate the viewer 1 has viewed when the viewer 1 has viewed the object 2 (viewing point = 1). When the viewer 1 has not viewed the object 2 (viewing point = 0), the instantaneous visual attention rate is 0. That is, the instantaneous visual attention rate is an instantaneous attention rate of the individual viewer 1 with respect to the object 2.

The determination unit 104 calculates an instantaneous visual feeling rate of the viewer 1 based on the viewing point that has been input at Step S301 and the feeling rate that has been calculated at Step S302, by the following expression (Step S305).

[Expression 9]

$$\text{Instantaneous visual feeling rate} = \text{Viewing point (1 or 0)} \times \text{Feeling rate} \ldots (9)$$

The instantaneous visual feeling rate is a value indicating with what degree of feeling rate the viewer has viewed when the viewer 1 has viewed the object 2 (viewing point = 1). When the viewer 1 has not viewed the object 2 (viewing point = 0), the instantaneous visual feeling rate is 0. That is, the instantaneous visual feeling rate is an instantaneous feeling rate of the individual viewer 1 with respect to the object 2.

The determination unit 104 calculates instantaneous visual object efficacy of the viewer 1 based on the viewing point that has been input at Step S301 and the viewing quality that has been calculated at Step S303, by the following expression (Step S306).

[Expression 10]

$$\text{Instantaneous visual object efficacy} = \text{Viewing point (1 or 0)} \times \text{Viewing quality} \ldots (10)$$

The instantaneous visual object efficacy is a value indicating with what degree of viewing quality (attention rate and feeling rate) the viewer 1 has viewed when the viewer 1 has viewed the object 2 (viewing point = 1). The instantaneous visual object efficacy indicates the degree of comprehensive impact of the viewer 1 with respect to the object 2. When the viewer 1 has not viewed the object 2 (viewing point = 0), the instantaneous visual object efficacy is 0. That is, the instantaneous viewing quality is instantaneous visual object efficacy of the individual viewer 1 with respect to the object 2.

The determination unit 104 outputs, for example, the instantaneous visual object efficacy that has been calculated at Step S306, as determination results, to the display unit 105 (Step S307). The determination unit 104 stores the determination results of, for example, the instantaneous visual object efficacy in the storage unit 103. In this case, the determination unit 104 also outputs and stores the attention rate, the feeling rate, the viewing quality, the instantaneous visual attention rate, and the instantaneous visual feeling rate, to the display unit 105 and in the storage unit 103, respectively.

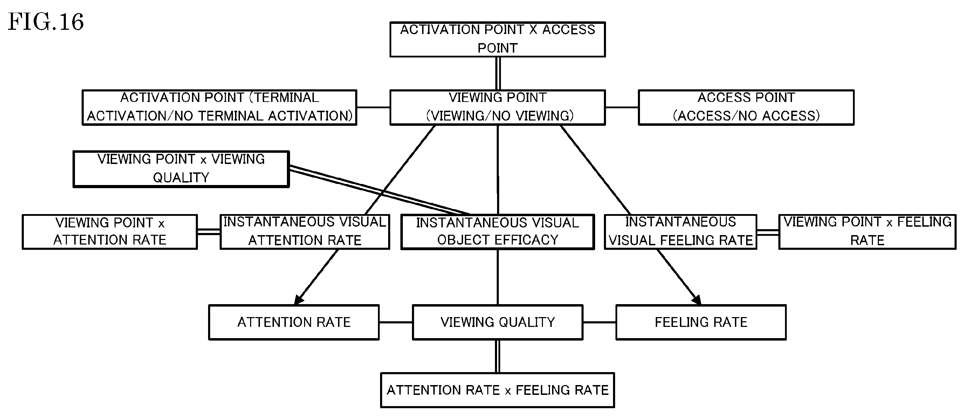

Fig. 16 is a diagram of connections between pieces of data and expressions upon the calculation of the instantaneous evaluation data of the individual viewer 1 with respect to the object 2 in Fig. 15(1). As illustrated in Fig. 16, the viewing point is acquired by multiplying the activation point by the access point. The viewing quality is acquired by multiplying the attention rate by the feeling rate. The instantaneous visual attention rate is acquired by multiplying the viewing point by the attention rate. The instantaneous visual feeling rate is acquired by multiplying the viewing point by the feeling rate. The instantaneous visual object efficacy is acquired by multiplying the viewing point by the viewing quality.

(Calculation processing for time course evaluation data of individual viewer 1 with respect to object 2: Fig. 15(2))

Next, calculation processing for time course evaluation data of the individual viewer 1 with respect to the object 2 in Fig. 15(2) by the determination unit 104 will be described. The determination unit 104 performs the processing illustrated in Fig. 3 every predetermined sampling time so as to calculate the time course evaluation data of the individual viewer 1 with respect to the object 2 in Fig. 15(2). That is, the determination unit 104 calculates the attention rate, the feeling rate, the viewing quality, the instantaneous visual attention rate, the instantaneous visual feeling rate, and the instantaneous visual object efficacy of the individual viewer 1 with respect to the object 2 in Fig. 15(1) every predetermined sampling. The determination unit 104 outputs and stores these pieces of time series data to the display unit 105 and the storage unit 103, respectively.

Accordingly, in a case where the object 2 is content, the time course evaluation data of the individual viewer 1 with respect to the content between start time and completion time of the content can be acquired. The display unit 105 to be described later graphs these pieces of time series data on an axis of time. Thus, the time course evaluation data of the individual viewer 1 with respect to the object 2 can be visualized.

Note that the determination unit 104 may calculate a maximum value of, for example, the degree of attention of the individual viewer 1 with respect to the object 2, during predetermined examination time between the start time and the completion time of the object 2. The determination unit 104 may also calculate a time integrated value and divides the time integrated value by the examination time so as to calculate an average value.

Connections between the pieces of data and the expressions upon the calculation of the time course evaluation data of the individual viewer 1 with respect to the object 2 in Fig. 15(2) have been already illustrated in Fig. 16. As illustrated in Fig. 16, the viewing point, the viewing quality, the instantaneous visual attention rate, the instantaneous visual feeling rate, and the instantaneous visual object efficacy are acquired every predetermined sampling time.

(Calculation processing for instantaneous evaluation data of all viewers 1 with respect to the same object 2: Fig. 15(3))

Next, calculation processing for the instantaneous evaluation data of all the viewers 1 with respect to the same object 2 in Fig. 15(3) by the determination unit 104 will be described. After the processing illustrated in Fig. 3, the determination unit 104 sums each of the attention rate, the feeling rate, the viewing quality, the instantaneous visual attention rate, the instantaneous visual feeling rate, and the instantaneous visual object efficacy of the individual viewer 1 with respect to the object 2 for the amount of all the viewers 1. The determination unit 104 calculates an average value and a maximum value. The determination unit 104 outputs and stores the average value and the maximum value to the display unit 105 and in the storage unit 103, respectively.

The determination unit 104 calculates an instantaneous viewing rate of all the viewers 1 with respect to the same object 2 based on the instantaneous audience share and the instantaneous access share that have been input at Step S301, by the following expression. The instantaneous viewing rate is a value acquired by multiplying the instantaneous audience share by the instantaneous access share. The instantaneous viewing rate indicates a ratio of viewers 1 who have actually viewed the object 2, to the total viewer number in all terminals to be examined 13.
[Expression 11]

Instantaneous viewing rate = Instantaneous audience share × Instantaneous access share ... (11)

In a case where the terminal to be examined 13 is a television receiver and the object 2 is a special TV program, the determination unit 104 calculates an instantaneous TV viewing rate based on the TV program instantaneous audience share and the TV instantaneous access share that have been input at Step S301, by the following expression. The instantaneous TV viewing rate indicates a ratio of viewers 1 whose gazes have accessed the special TV program displayed on the television receiver in which a special channel has been selected, to all television receivers to be examined.

[Expression 12]

Instantaneous TV viewing rate = TV program instantaneous audience share × TV instantaneous

access share ... (12)

The determination unit 104 calculates the instantaneous visual attention rate of all the viewers 1 with respect to the same object 2 based on the instantaneous viewing rate and an average value of the attention rate (average value of attention rates in all the viewers 1), by the following expression. The instantaneous visual attention rate indicates an instantaneous attention rate of all the viewers 1 with respect to the same object 2 in all the terminals to be examined 13. [Expression 13]

Instantaneous visual attention rate = Instantaneous viewing rate × (Average value of attention

rate) ... (13)

The determination unit 104 calculates the instantaneous visual feeling rate of all the viewers 1 with respect to the same object 2 based on the instantaneous viewing rate and an average value of the feeling rate (average value of feeling rates of all the viewers 1), by the following expression. The instantaneous visual feeling rate indicates an instantaneous feeling rate of all the viewers 1 with respect to the same object 2 in all the terminals to be examined 13. [Expression 14]

Instantaneous visual feeling rate = Instantaneous viewing rate × (Average value of feeling

rate) ... (14)

In a case where the object 2 is content, the instantaneous visual feeling rate of all the viewers 1 with respect to the same object 2 is used for determining whether a feeling of the viewer 1 who has viewed the content has appeared as intended by the content maker. The instantaneous visual feeling rate is also used in order to determine whether the degree of the feeling has a high level.

The determination unit 104 calculates the instantaneous visual object efficacy of all the viewers 1 with respect to the same object 2 based on the instantaneous viewing rate and an average value of the viewing quality (average value of viewing qualities of all the viewers 1), by the following expression. The instantaneous visual object efficacy indicates an instantaneous viewing quality of all the viewers 1 with respect to the same object 2 in all the terminals to be examined 13. More specifically, the instantaneous visual object efficacy indicates with what degree of attention and with what degree of feeling all the viewers 1 have viewed. [Expression 15]

Instantaneous visual object efficacy = Instantaneous viewing rate × (Average value of viewing

quality)

= (Instantaneous audience share × Instantaneous access share) × (Average value of attention

rate × Average value of feeling rate) ... (15)

The instantaneous visual object efficacy of all the viewers 1 with respect to the same object 2 indicates what degree of impact the object 2 has. In a case where the terminal to be examined 13 is a television receiver, the instantaneous visual object efficacy can be used for a new evaluation criterion instead of the audience share.

The instantaneous visual object efficacy covers all the terminals to be examined 13. In a case of a market survey based on a premise that content of a special object 2 in a special place is viewed, since all the terminals to be examined 13 have displayed the same content, the instantaneous viewing rate in Expression (15) above (refer to Expression (11)) is 1 (100%).

Each in a case where the object 2 is a WEB screen and it is premised that content on the WEB screen is viewed, in a case where the object 2 is a piece of printed matter (electronic book in the present example) and it is premised that the electronic book is read, in a case where the object 2 is a display board, such as a poster or a sign board, and it is premised that the display board is viewed, or in a case where the object 2 is a commodity and it is premised that the commodity is viewed, the instantaneous viewing rate in Expression (15) (refer to Expression (11)), is also 1(100%).

The determination unit 104 outputs and stores the instantaneous viewing rate, the instantaneous visual attention rate, the instantaneous visual feeling rate, and the instantaneous visual object efficacy, to the display unit 105 and in the storage unit 103, respectively.

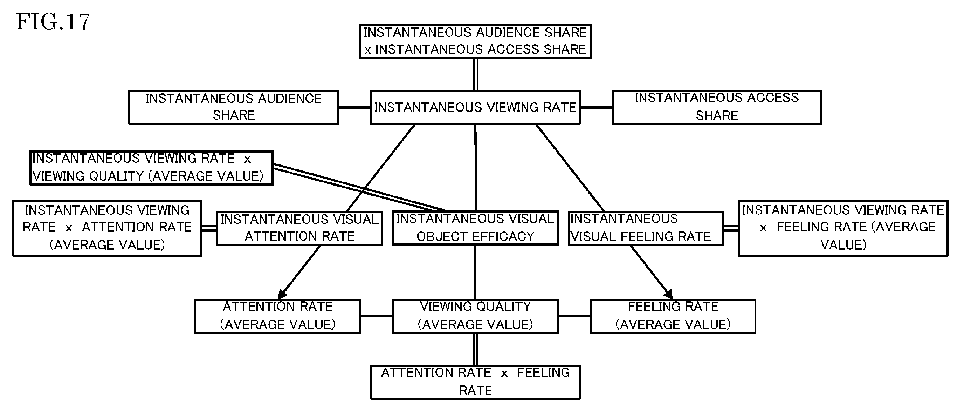

Fig. 17 is a diagram of connections between the pieces of data and expressions upon the calculation of the instantaneous evaluation data of all the viewers 1 with respect to the same object 2 in Fig. 15(3). As illustrated in Fig. 17, the instantaneous viewing rate is acquired by multiplying the instantaneous audience share by the instantaneous access share. The average value of the viewing quality is acquired by multiplying the average value of the attention rate by the average value of the feeling rate. The instantaneous visual attention rate is acquired by multiplying the instantaneous viewing rate by the average value of the attention rate. The instantaneous visual feeling rate is acquired by multiplying the instantaneous viewing rate by the average value of the feeling rate. The instantaneous visual object efficacy is acquired by multiplying the instantaneous viewing rate by the average value of the viewing quality.

(Calculation processing for time course evaluation data of all viewers 1 with respect to the same object 2: Fig. 15(4))

Next, calculation processing for time course evaluation data of all the viewers 1 with respect to the same object 2 in Fig. 15(4) by the determination unit 104 will be described. The determination unit 104 performs the calculation processing for the instantaneous evaluation data of all the viewers 1 with respect to the same object 2 in Fig. 15(3) every predetermined sampling time so as to calculate the time course evaluation data of all the viewers 1 with respect to the same object 2 in Fig. 15(4). That is, the determination unit 104 calculates the attention rate, the feeling rate, the viewing quality, the instantaneous viewing rate, the instantaneous visual attention rate, the instantaneous visual feeling rate, and the instantaneous visual object efficacy of all the viewers 1 with respect to the same object 2 in Fig. 15(3) every predetermined sampling. The determination unit 104 outputs and stores these pieces of time series data to the display unit 105 and in the storage unit 103, respectively. The determination unit 104 sums these pieces of time series data during predetermined examination time so as to calculate an average value and a maximum value. The determination unit 104 outputs and stores the values to the display unit 105 and in the storage unit 103, respectively.

For example, the determination unit 104 calculates visual object efficacy of all the viewers 1 with respect to the same object 2 (average value of the instantaneous visual object efficacy) based on the instantaneous visual object efficacy of all the viewers 1 with respect to the same object 2 in Fig. 15(3) and the predetermined examination time, by the following expression. The visual object efficacy of all the viewers 1 with respect to the same object 2 is a value acquired by statistically calculating the instantaneous visual object efficacy of all the viewers 1 with respect to the same object 2 in Fig. 15(3) during the predetermined examination time. The visual object efficacy is an average value of the instantaneous visual object efficacy during the predetermined examination time.

[Expression 16]

$$\text{Visual object efficacy} = \frac{\int_{t=0}^{t=T} (\text{Instantaneous visual object efficacy})\, dt}{T} \quad \dots (16)$$

Here, T represents the predetermined examination time, and t represents elapsed time. In a case where the object 2 is content, T represents running time. In a case where the object 2 is a TV program, T represents broadcast time. In a case where the object 2 is a WEB screen, T represents display time. In a case where the object 2 is a piece of printed matter (electronic book in the present example), T represents time during which the electronic book has been viewed. In a case where the object 2 is a display board, such as a poster or a sign board, or a commodity, T represents time during which the viewer 1 has stayed at a place at which the viewer 1 can view the display board or the commodity.

Note that, for the attention rate, the feeling rate, the viewing quality, the instantaneous viewing rate, the instantaneous visual attention rate, and the instantaneous visual feeling rate, the determination unit 104 also calculates average values based on these pieces of data of all the viewers 1 with respect to the same object 2 in Fig. 15(3), and the predetermined examination time as in Expression (16).

The determination unit 104 outputs and stores data of, for example, the visual object efficacy of all the viewers 1 with respect to the same object 2 to the display unit 105 and in the storage unit 103, respectively.

Accordingly, in a case where the object 2 is content, the time course evaluation data of all the viewers 1 with respect to the same object 2 can be acquired as a statistic value during the predetermined examination time between start time and completion time of the content.

Connections between the pieces of data and the expressions upon the calculation of the time course evaluation data of all the viewers 1 with respect to the same object 2 in Fig. 15(4) have been already illustrated in Fig. 17. In the time course evaluation data of all the viewers 1 with respect to the same object 2 in Fig. 15(4), an average value of the instantaneous viewing rate, an average value of the attention rate, an average value of the feeling rate, an average value of the instantaneous visual attention rate, an average value of the instantaneous visual feeling rate, and an average value of the instantaneous visual object efficacy are calculated during the predetermined examination time.

(Display unit)

The display unit 105 inputs the determination results from the determination unit 104, and displays the determination results on a display device, such as a display.

As described above, according to the visual object efficacy measuring device 100 of the embodiment of the present invention, the determination unit 104 has been made to calculate the viewing quality in which the emotion-and-feeling including the emotion and the feeling combined has been converted into a numerical form, for the individual viewer 1 based on the degree of attention and the degree of feeling with respect to the object 2 as in Fig. 15(1). The determination unit 104 has been made to calculate, for the individual viewer 1, the instantaneous visual attention rate, the instantaneous visual feeling rate, and the instantaneous visual object efficacy indicating with what degree of attention rate, what degree of feeling rate, and what degree of viewing quality the viewer 1 has viewed, respectively, when the viewer 1 has viewed (accessed) the object 2.

According to the visual object efficacy measuring device 100 of the embodiment of the present invention, the determination unit 104 has been made to calculate, for the individual viewer 1, the attention rate, the feeling rate, the viewing quality, the instantaneous visual attention rate, the instantaneous visual feeling rate, and the instantaneous visual object efficacy with respect to the object 2, in time series during the predetermined sampling time as in Fig. 15(2).

Accordingly, for example, the instantaneous visual object efficacy indicating the degree of physiological response including the degree of attention and the degree of feeling with respect to the object 2 can be displayed in time series on a screen as a determination value for evaluating the object 2, for the individual viewer 1. Therefore, the object 2, such as content, viewed by the viewer 1, can be further correctly evaluated.

According to the visual object efficacy measuring device 100 of the embodiment of the present invention, the determination unit 104 has made to calculate the average value and the maximum value of all the viewers 1 with respect to the same object 2, for each of the attention rate, the feeling rate, the viewing quality, the instantaneous visual attention rate, the instantaneous visual feeling rate, and the instantaneous visual object efficacy of the individual viewer 1 with respect to the object 2 as in Fig. 15(3). The determination unit 104 has been made to calculate the instantaneous viewing rate indicating a ratio of the viewers 1 who have viewed the object 2 to all the terminals to be examined 13, by multiplying the instantaneous audience share by the instantaneous access share. The determination unit 104 has been made to calculate the instantaneous visual attention rate, the instantaneous visual feeling rate, and the instantaneous visual object efficacy indicating the instantaneous attention rate, the instantaneous feeling rate, and the instantaneous viewing quality of all the viewers 1 with respect to the same object 2, in all the terminals to be examined 13, by multiplying the instantaneous viewing rate by the average value of the attention rate, the average value of the feeling rate, and the average of the viewing quality, respectively.

Accordingly, for all the viewers 1, for example, the instantaneous visual object efficacy indicating the degree of physiological response including the degree of attention and the degree of feeling with respect to the object 2 can be displayed in time series on a screen as a determination value for evaluating the object 2. Therefore, the object 2, such as content, viewed by all the viewers 1, can be further correctly evaluated.

According to the visual object efficacy measuring device 100 of the embodiment of the present invention, as in Fig. 15(4), the determination unit 104 has been made to calculate the attention rate, the feeling rate, the viewing quality, the instantaneous viewing rate, the instantaneous visual attention rate, the instantaneous visual feeling rate, and the instantaneous visual object efficacy of all the viewers 1 with respect to the same object 2 in time series during the predetermined sampling. The determination unit 104 has been made to sum these pieces of time series data during the predetermined examination time, and calculate the average value and the maximum value.

Accordingly, for all the viewers 1, for example, the instantaneous visual object efficacy indicating the degree of physiological response including the degree of attention and the degree of feeling with respect to the object 2, can be displayed on a screen as data during the predetermined examination time, as a determination value for evaluating the object 2. Therefore, the object 2, such as content, viewed by all the viewers 1, can be further correctly evaluated.

For example, in a case where the object 2 is a TV program, conventionally, the object 2 is evaluated only based on an audience share that is a ratio of the number of television receivers on which the TV program has been displayed. According to the visual object efficacy measuring device 100 of the embodiment of the present invention, the object 2 that is the TV program has been made to be evaluated based on, for example, the instantaneous visual object efficacy. Thus, the instantaneous access share indicating a ratio of the number of viewers 1 each whose gazes have accessed the region of the TV program, the instantaneous viewing rate indicating a ratio of the viewers 1 who have viewed the TV program, and the viewing quality including the degree of attention and the degree of feeling with respect to the TV program, can be added. Therefore, further correct evaluation can be achieved.

In a case where the object 2 is a TV CM, conventionally, the object 2 is also evaluated only based on a ratio of the number of television receivers that is an audience share. Evaluation for the CM receives an effect due to, for example, content of a program just before the CM, or a field of interest of a viewer 1 at the point in time. Therefore, it is difficult to evaluate the CM, correctly. According to the visual object efficacy measuring device 100 of the embodiment of the present invention, the object 2 that is a CM has been made to be evaluated based on, for example, the visual object efficacy. Thus, the viewing quality including the degree of attention and the degree of feeling with respect to the CM can be added. Furthermore, the degree of impact of the viewer 1 with respect to the CM itself can be determined based on, for example, the instantaneous visual object efficacy. Therefore, further correct evaluation can be achieved including various effects, such as content of a program just before and a field of interest of the viewer 1 at the point in time.

(Types of object 2 and pieces of data)

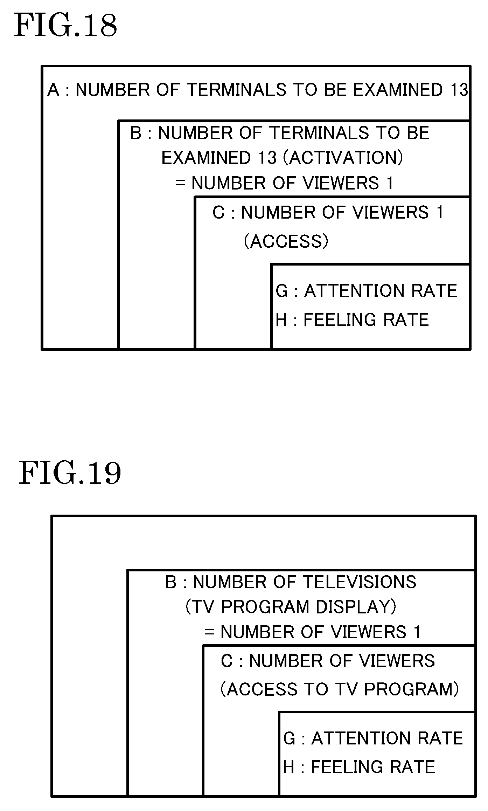

Next, relationships between types of object 2 and each of pieces of data handled by the visual object efficacy measuring device 100 will be described. Fig. 18 is a diagram for describing relationships between the pieces of data in the instantaneous evaluation data of all the viewers 1 with respect to the same object 2 in Fig. 15(3) in a case where the object 2 is shared. The number of terminals to be examined 13 previously set (the total number of terminals to be examined) is defined as A. The instantaneous visual object activation number that is the number of terminals to be examined 13 on which the object 2 has been activated (displayed) and the number of viewers 1 (total viewer number) are defined as B. In this case, the instantaneous visual object activation number that is the number of terminals to be examined 13, and the number of viewers 1 (total viewer number) are defined as the same. The instantaneous access viewer number that is the number of viewers 1 whose gazes have accessed the region of the object 2, is defined as C. The attention rate (average value of attention rates of all the viewers 1) is defined as G. The feeling rate (average value of feeling rates of all the viewers 1) is defined as H. A magnitude correlation satisfies $A > B > C$.

The instantaneous audience share D is acquired by dividing the instantaneous visual object activation number B by the total number of terminals to be examined A ($D = B/A$). The instantaneous audience share D is a ratio of terminals to be examined 13 on which the object 2 has been activated, to the total number of terminals to be examined A. The instantaneous audience share D corresponds to Expression (2) above. The instantaneous access share E is acquired by dividing the instantaneous access viewer number C by the total viewer number B (instantaneous visual object activation number B) ($E = C/B$). The instantaneous access share E is a ratio of the instantaneous access viewer number including the number of viewers 1 whose gazes have accessed the region of the object 2, to the total viewer number B. The instantaneous access share E corresponds to Expression (4) above.

The instantaneous viewing rate F is acquired by multiplying the instantaneous audience share D by the instantaneous access share E ($F = D \times E = B/A \times C/B = C/A$). The instantaneous viewing rate F corresponds to Expression (11) above. The instantaneous viewing rate F is also a ratio of the instantaneous access viewer number C to the total number of terminals to be examined A. The viewing quality (average value of viewing qualities in all the viewers 1) I is acquired by multiplying the attention rate G by the feeling rate H ($I = G \times H$). The viewing quality I is a value indicating with what degree of attention and what degree of feeling the viewer 1 has viewed when the viewer 1 has viewed the object 2. The viewing quality corresponds to Expression (7) above. The instantaneous visual attention rate J is acquired by multiplying the attention rate G by the instantaneous viewing rate F ($J = G \times F$). The instantaneous visual attention rate J is a value indicating with what degree of attention the viewer 1 has viewed when the viewer has viewed the object 2, in a case of all the terminals to be examined 13 and all the viewers 1. The instantaneous visual attention rate J corresponds to Expression (13) above. A value of the instantaneous visual attention rate J decreases when a small number of viewers 1 have accessed the object 2 and the instantaneous viewing rate F is low even in a case where the attention rate G is high.

The instantaneous visual feeling rate K is acquired by multiplying the feeling rate H by the instantaneous viewing rate F ($K = H \times F$). The instantaneous visual feeling rate K is a value indicating with what degree of feeling the viewer 1 has viewed when the viewer 1 has viewed the object 2 in a case of all the terminals to be examined 13 and all the viewers 1. The instantaneous visual feeling rate K corresponds to Expression (14) above. A value of the instantaneous visual feeling rate K decreases when a small number of viewers 1 have accessed the object 2 and the instantaneous viewing rate F is low even in a case where the feeling rate H is high. The instantaneous visual object efficacy L is acquired by multiplying the instantaneous viewing rate F by the viewing quality I ($L = F \times I$). The instantaneous visual object efficacy L is a value indicating with what degree of attention and what degree of feeling the viewer 1 has viewed when the viewer 1 has viewed the object 2 in a case of all the terminals to be examined 13 and all the viewers 1. The instantaneous visual object efficacy L corresponds to Expression (15) above. A value of the instantaneous visual object efficacy L decreases when a small number of viewers 1 have accessed the object 2 and the instantaneous viewing rate F is low even in a case where the viewing quality I is high.

Fig. 19 is a diagram for describing relationships between each of pieces of data in a case where the object 2 is a TV program. The number of television receivers to be examined previously set is defined as A. The number of television receivers on which the TV program has been displayed, and the number of viewers 1 are defined as B. The number of viewers 1 whose gazes each have accessed a region of the TV program, is defined as C. The attention rate (average value of attention rates in all the viewers 1) is defined as G. The feeling rate (average value of feeling rates in all the viewers 1) is defined as H. In this case, the instantaneous audience share D, the instantaneous access share E, the instantaneous viewing rate F, the viewing quality I, the instantaneous visual attention rate J, the instantaneous visual feeling rate K, and the instantaneous visual object efficacy L are acquired as in Fig. 18.

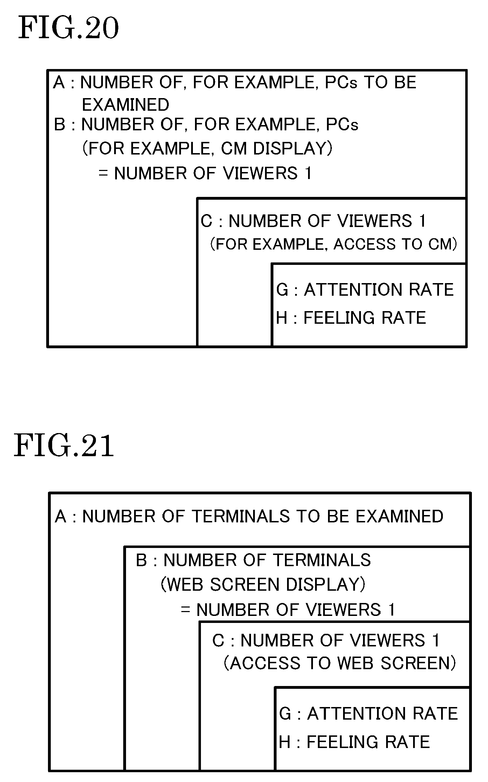

Fig. 20 is a diagram for describing relationships between each of pieces of data in a case where the object 2 is, for example, a CM displayed on, for example, a personal computer or a mobile terminal. The number of, for example, personal computers to be examined previously set, is defined as A. The number of, for example, personal computers on which, for example, the CM has been displayed, and the number of viewers 1 are defined as B. The number of viewers 1 whose gazes each have accessed a region of, for example, the special CM, is defined as C. The attention rate (average value of attention rates in all the viewers 1) is defined as G. The feeling rate (average value of feeling rates in all the viewers 1) is defined as H. A = B is satisfied. In this case, the instantaneous audience share D becomes 1 (D = B/A = 1/1 =1). As in Fig. 18, the instantaneous access share E, the instantaneous viewing rate F, the viewing quality I, the instantaneous visual attention rate J, the instantaneous visual feeling rate K, and the instantaneous visual object efficacy L are acquired.

Fig. 21 is a diagram for describing relationships between each of pieces of data in a case where the object 2 is a WEB screen. The number of terminals to be examined previously set is defined as A. The number of terminals to be examined on which the special WEB screen has displayed, and the number of viewers 1 are defined as B. The number of terminals to be examined on which the number of viewers 1 whose gazes each have accessed a region of the special WEB screen, is defined as C. The attention rate (average value of attention rates in all the viewers 1) is defined as G. The feeling rate (average value of feeling rates in all the viewers 1) is defined H. In this case, as in Fig. 18, the instantaneous audience share (instantaneous display rate) D, the instantaneous access share (instantaneous WEB access share) E, the instantaneous viewing rate (instantaneous WEB viewing rate) F, the viewing quality I, the instantaneous visual attention rate J, the instantaneous visual feeling rate K, and the instantaneous visual object efficacy L are acquired.

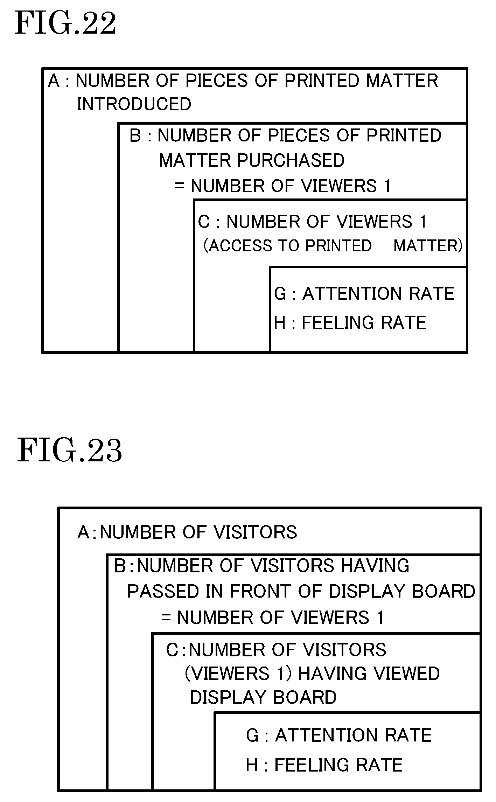

Fig. 22 is a diagram for describing relationships between each of pieces of data in a case where the object 2 is a piece of printed matter (electronic book in the present example). The number of pieces of printed matter previously set (the number of pieces of printed matter introduced) is defined as A. The number of pieces of printed matter that have been purchased and the number of viewers 1 are defined as B. The number of viewers 1 whose gazes each have accessed a region, for example, a letter in the printed matter, is defined as C. The attention rate (average value of attention rates in all the viewers 1) is defined as G. The feeling rate (average value of feeling rates in all the viewers 1) is defined as H. In this case, as in Fig. 18, the instantaneous audience share (instantaneous purchasing rate) D, the instantaneous access share (instantaneous purchase reading rate) E, the instantaneous viewing rate (instantaneous introduced printed matter reading rate) F, the viewing quality I, the instantaneous visual attention rate J, the instantaneous visual feeling rate K, and the instantaneous visual object efficacy L are acquired.

Fig. 23 is a diagram for describing relationships between each of pieces of data in a case where the object 2 is a display board, such as a poster or a sign board. The number of visitors who have visited, for example, a site at which the display board has been disposed, is defined as A. The number of visitors who have passed in front of the display board, and the number of viewers 1 are defined as B. The number of viewers 1 who have visited and viewed the display board, is defined as C. The attention rate (average value of attention rates in all the viewers 1) is defined G. The feeling rate (average value of feeling rates in all the viewers 1) is defined as H. A and B are numbers that have been counted by detecting the visitors by a sensor. In this case, as in Fig. 18, the instantaneous audience share (instantaneous visitor passing rate) D, the instantaneous access share (instantaneous passer access share) E, the instantaneous viewing rate (instantaneous visitor access rate) F, the viewing quality I, the instantaneous visual attention rate J, the instantaneous visual feeling rate K, and the instantaneous visual object efficacy L are acquired.

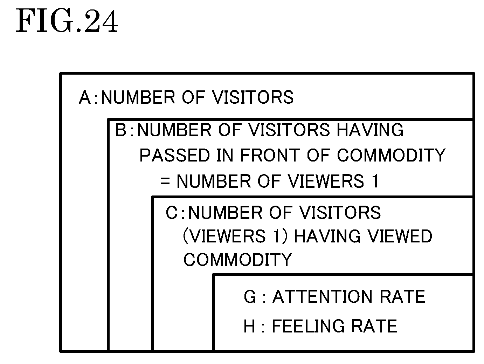

Fig. 24 is a diagram for describing relationships between each of pieces of data in a case where the object 2 is a commodity. The number of visitors who have visited, for example, a site at which the commodity has been disposed, is defined A. The number of visitors who have passed in front of the commodity, and the number of viewers 1 are defined as B. The number of viewers 1 who have visited and viewed the commodity, is defined as C. The attention rate (average value of attention rates in all the viewers 1) is defined as G. The feeling rate (average value of feeling rates in all the viewers 1) is defined as H. A and B are numbers that have been counted by detecting the visitors by a sensor. In this case, as in Fig. 18, the instantaneous audience share (instantaneous visitor passing rate) D, the instantaneous access share (instantaneous passer access share) E, the instantaneous viewing rate (instantaneous visitor access rate) F, the viewing quality I, the instantaneous visual attention rate J, the instantaneous visual feeling rate K, and the instantaneous visual object efficacy L are acquired.

(Visual object efficacy measuring system/Second Embodiment)

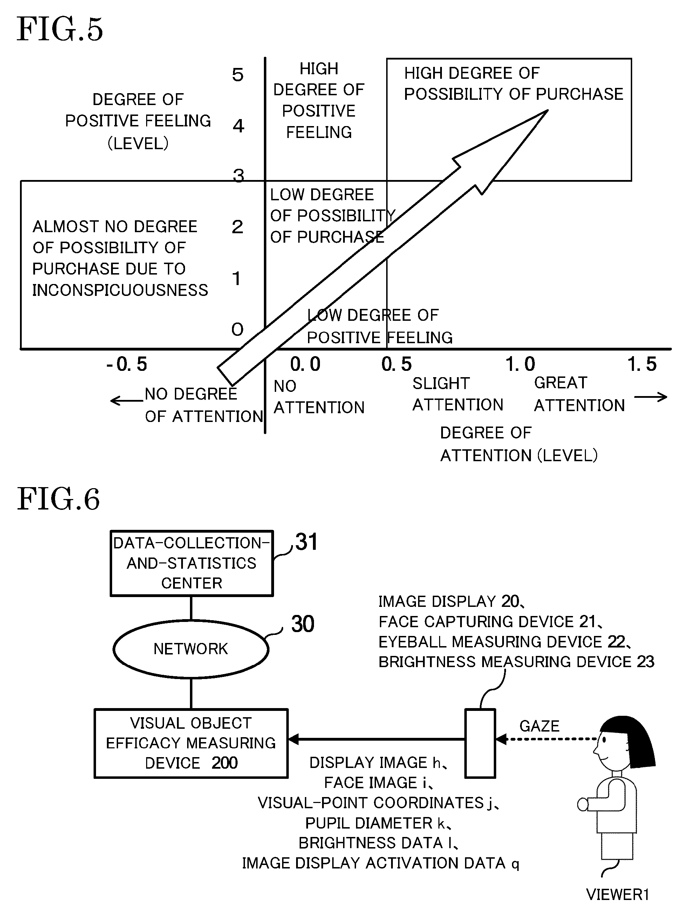

Next, a configuration and each configuration unit of a visual object efficacy measuring system including a visual object efficacy measuring device according to a second embodiment of the present invention will be described. Fig. 6 is a schematic diagram of the configuration of the visual object efficacy measuring system including the visual object efficacy measuring device according to the second embodiment of the present invention. The visual object efficacy measuring system includes an image display 20, a face capturing device 21, an eyeball measuring device 22, a brightness measuring device 23, a visual object efficacy measuring device 200, and a data-collection-and-statistics center 31. The visual object efficacy measuring device 200 and the data-collection-and-statistics center 31 are coupled through a network 30, such as the Internet.

A viewer 1 faces a gaze toward the image display 20 in order to view content displayed on the image display 20, the content being an object 2. As data of the content displayed on the image display 20, response evocating information for causing the viewer 1 to respond, such as a character string, a figure, a symbol, a picture, a photograph, or a moving image, is displayed. The data includes response object information, such as the character string, the figure, the symbol, the picture, the photograph, or the moving image, to be an object to which the viewer 1 responds.

The face capturing device 21, the eyeball measuring device 22, and the brightness measuring device 23 are disposed near the image display 20. The image display 20 generates image display activation data q indicating whether the image display 20 has been activated, and outputs the image display activation data q and a display image h that has been displayed on a screen, to the visual object efficacy measuring device 200. The image display activation data q is the same as the terminal-to-be-examined activation data s output by the terminal to be examined 13 illustrated in Fig. 1. The face capturing device 21 captures a face of the viewer 1, and outputs a face image i to the visual object efficacy measuring device 200. The face image i is used for analyzing a movement and an expression of a face of the viewer 1. The degree of feeling with respect to the display image h displayed on the image display 20, is calculated based on the face image i.

The eyeball measuring device 22 captures, for example, an eyeball of the viewer 1, measures information on the eye ball based on an image that has been captured, and outputs visual-point coordinates j and a pupil diameter k of the information on the eyeball to the visual object efficacy measuring device 200. The visual-point coordinates j indicate coordinates of a visual-point position accessed by a gaze of the viewer 1 in a case where the display image h displayed on the image display 20 has been expressed in a plane including x and y axes. Based on the visual-point coordinates j, it is determined whether the display image h displayed on the image display 20 has been accessed. Based on the pupil diameter k, the degree of attention with respect to the display image h displayed on the image display 20 is calculated.

The brightness measuring device 23 measures and outputs brightness of a display screen of the image display 20, to the visual object efficacy measuring device 200, as brightness data l. The brightness data l is used for correcting the pupil diameter k of the viewer 1.

Note that, in a case where the face capturing device 21 captures the faces of a plurality of viewers 1, or in a case where the eyeball measuring device 22 captures the eyeballs of the plurality of viewers 1, the face capturing device 21 outputs the face image i (may be an image including the faces of the plurality of viewers 1) and the eyeball measuring device 22 outputs the visual-point coordinates j and the pupil diameter k, for each of the plurality of viewers 1.

The visual object efficacy measuring device 200 inputs the image display activation data q and the display image h from the image display 20, the face image i from the face capturing device 21, the visual-point coordinates j and the pupil diameter k from the eyeball measuring device 22, the brightness data l from the brightness measuring device 23. Based on these pieces of data, the visual object efficacy measuring device 200 generates analysis results, such as the degree of attention and the degree of feeling with respect to the display image h. Based on the analysis results, the visual object efficacy measuring device 200 generates determination results, such as an attention rate, a feeling rate, and a viewing quality with respect to the display image h. The visual object efficacy measuring device 200 transmits the pieces of data such as the display image h and the face image i, the analysis results, and the determination results, to the data-collection-and-statistics center 31 through the network 30.

The data-collection-and-statistics center 31 receives the data, such as the display image h and the face image i, the analysis results, and the determination results through the network 30 from the visual object efficacy measuring device 200, and performs statistical processing.

Note that, the data-collection-and-statistics center 31 may transmit a result of the statistical processing to the visual object efficacy measuring device 200 through the network 30. The visual object efficacy measuring device 200 may receive the result of the statistical processing from the data-collection-and-statistics center 31 through the network 30, and display the result on a screen. The data-collection-and-statistics center 31 may be made to generate the analysis results and the determination results instead of the visual object efficacy measuring device 200.

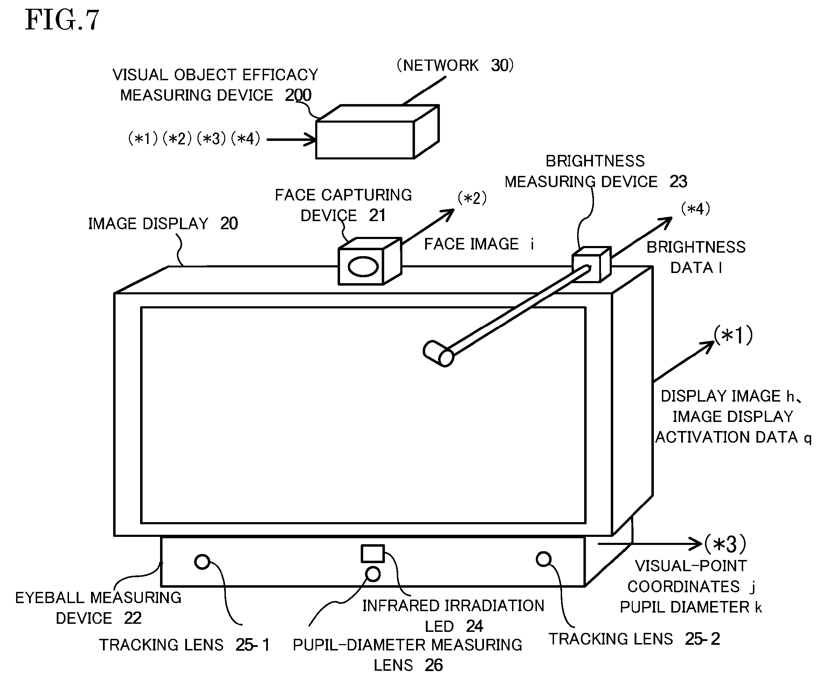

Fig. 7 is a view for describing external appearances of the image display 20, the face capturing device 21, the eyeball measuring device 22, the brightness measuring device 23, and the visual object efficacy measuring device 200 illustrated in Fig. 6. As illustrated in Fig. 7, the face capturing device 21 is disposed near the center of an upper surface of the image display 20. The brightness measuring device 23 is disposed on a corner of the upper surface of the image display 20. The brightness measuring device 23 includes a sensor disposed on a head end thereof and facing the display screen of the image display 20, in order to measure the brightness of the display screen of the image display 20.

The eyeball measuring device 22 is disposed on a lower surface of the image display 20 so as to be loaded with the image display 20. The eyeball measuring device 22 includes an infrared irradiation LED 24, tracking lenses 25-1 and 25-2, and a pupil-diameter measuring lens 26. The eyeball measuring device 22 has a function for capturing a predetermined part including the eye of the viewer 1, a function for capturing the eyeball of the viewer 1, a function for tracking a position of the eye of the viewer 1 (the left eye or the right eye previously set) by analyzing an image, including the eye, that has been captured, and a function for measuring the visual-point coordinates j and the pupil diameter k of the viewer 1 by analyzing the eyeball image that has been captured.

More specifically, the eyeball measuring device 22 captures the eye of the viewer 1 by using two lenses including the tracking lenses 25-1 and 25-2. Based on the image of the eye of the viewer 1 that has been captured, the eyeball measuring device 22 triangulates the eye of the viewer 1, measures a direction and a distance of a position of the eye, and tracks the position of the eye. The eyeball measuring device 22 faces the tracking lenses 25-1 and 25-2 toward the eye in accordance with the position of the eye that has been tracked. The eyeball measuring device 22 captures the eyeball of the viewer 1 using the pupil-diameter measuring lens 26. Based on the eyeball image of the viewer 1 that has been captured, the eyeball measuring device 22 measures a pupil diameter of the eye of the viewer 1. The eyeball measuring device 22 corrects the pupil diameter that has been measured, by a distance, and then acquires the pupil diameter k after the correction.

The eyeball measuring device 22 irradiates infrared rays from the infrared irradiation LED 24 to the eye of the viewer 1. The eyeball measuring device 22 captures a line of cornea reflection so as to measure the visual-point coordinates j of the viewer 1. The infrared rays irradiated from the infrared irradiation LED 24, is used as lighting for measuring the pupil diameter of the eye of the viewer 1. The infrared rays are light beams in which the line of cornea reflection of the eye of the viewer 1 can be captured.

Note that, for example, processing for tracking the eye of the viewer 1 and processing for measuring the pupil diameter k by the eyeball measuring device 22 have been known. Thus, the detailed descriptions will be omitted here. A method for measuring the visual-point coordinates j has been known. Another method may be used.

As described above, the visual-point coordinates j to be measured indicates the visual-point position in a case where the display image h displayed on the image display 20 has been expressed in the plane including the x and y axes. In a case where the gaze of the viewer 1 is in the display image h, the visual-point coordinates are expressed by the coordinate values. In a case where the gaze of the viewer 1 is out of the display image h, the visual-point coordinates are expressed by numerical values from which it can be determined that the visual-point coordinates are out of the display image h. Therefore, the visual object efficacy measuring device 200 can determine whether the gaze of the viewer 1 is in or out of the display image h, based on the visual-point coordinates j. That is, the visual object efficacy measuring device 200 can generate an access point and a viewing point included in visual-point access/no visual-point access information indicating whether the gaze of the viewer 1 has accessed the display image h.

The image display activation data q generated by the image display 20, the display image h displayed on the image display 20, the face image i captured by the face capturing device 21, the visual-point coordinates j and the pupil diameter k measured by the eyeball measuring device 22, the brightness data l measured by the brightness measuring device 23 are output to the visual object efficacy measuring device 200. In a case of a plurality of viewers 1, a face image i (may be an image including faces of the plurality of viewers 1), the visual-point coordinates j and a pupil diameter k of each of the plurality of viewers 1 are output.

Note that, in Fig. 7, the image display 20, the face capturing device 21, the eyeball measuring device 22, and the brightness measuring device 23 have been given as individual devices. A configuration of a device integrally includes these devices can be applied. The display image h, the face image i, the visual-point coordinates j, the pupil diameter k, the brightness data l, and the image display activation data q may be output to the visual object efficacy measuring device 200 through a wired cable. Alternatively, the pieces of data may be transmitted to the visual object efficacy measuring device 200 through wireless communication.

(Visual object efficacy measuring device 200)

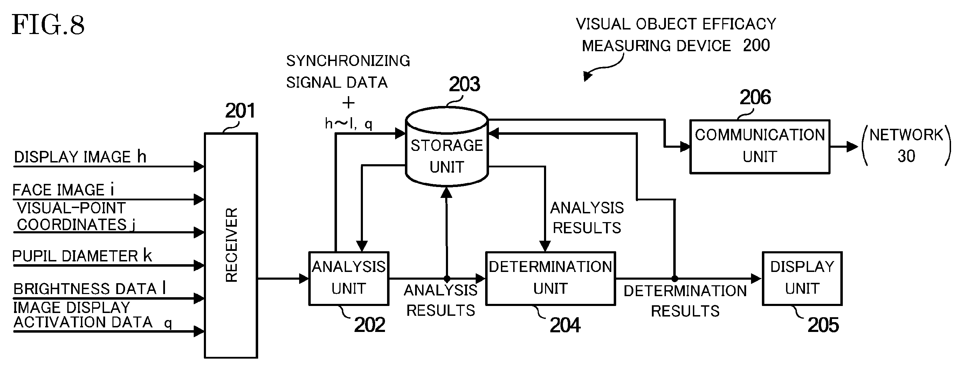

Next, a configuration and each configuration unit of the visual object efficacy measuring device 200 illustrated in Fig. 6 will be described. Fig. 8 is a block diagram of the configuration of the visual object efficacy measuring device 200 according to the second embodiment of the present invention. The visual object efficacy measuring device 200 includes a receiver 201, an analysis unit 202, a storage unit 203, a determination unit 204, a display unit 205, and a communication unit 206.

(Receiver)

The receiver 201 respectively inputs the image display activation data q and the display image h from the image display 20, the face image i from the face capturing device 21, the visual-point coordinates j and the pupil diameter k from the eyeball measuring device 22, and the brightness data I from the brightness measuring device 23. The receiver 201 synchronizes all the pieces of data that have been input. The receiver 201 outputs each of the pieces of data to the analysis unit 202 at predetermined time intervals or in accordance with an instruction from the analysis unit 202.

Note that, in order to synchronize the display image h, the face image i, the visual-point coordinates j, the pupil diameter k, the brightness data l, and the image display activation data q for the visual object efficacy measuring system illustrated in Fig. 6, each of the pieces of data may include time of a clock as a synchronizing signal in a manner similar to the visual object efficacy measuring system illustrated in Fig. 1. The viewer 1 may press a switch, not illustrated in Figs. 6 and 7, so as to start the synchronization.

(Analysis unit)

The analysis unit 202 inputs the display image h, the face image i, the visual-point coordinates j, the pupil diameter k, the brightness data l, and the image display activation data q from the receiver 201 at the predetermined time intervals or by the instruction to the receiver 201. The analysis unit 202 adds a synchronizing signal to the pieces of data h to l, and q, and stores the pieces of data h to l, and q with the synchronizing signal in the storage unit 203. As necessary, the analysis unit 202 reads the pieces of data h to l, and q that have been stored in the storage unit 203, uses the pieces of data h to l, and q that have been read or the pieces of data h to l, and q that have been input for analysis, generates analysis results, stores the analysis result that has been generated in the storage unit 203, and outputs the analysis results to the determination unit 204.

More specifically, the analysis unit 202 generates, for example, the degree of attention, the degree of feeling, the access point, the viewing point, an instantaneous audience share, an instantaneous access share with respect to the display image h, as analysis results, in a manner similar to the analysis unit 102 illustrated in Fig. 2.

For example, the analysis unit 202 corrects the pupil diameter k by removing a pupil diameter changing part that corresponds to brightness of the object 2 to be a visual object, from the pupil diameter k, using the brightness data l. The analysis unit 202 calculates the degree of attention with respect to the display image h based on the pupil diameter after the correction.

The analysis unit 202 grasps each of parts of features of the face of the viewer 1 (for example, the eye, a lip, and an eyebrow) by an image analysis technique using the face image i so as to measure a variation of each of the parts. In a manner similar to the analysis unit 102, the analysis unit 202 previously measures values when the viewer 1 is in a normal state and values when the parts of the face of the viewer 1 move to a maximum extent (maximum values). The analysis unit 202 calculates the degree of feeling by analyzing an expression of the face, based on a ratio of each of variations when the parts are measured, to each of the maximum values. The degree of feeling is calculated for each of feelings of six basic feelings (pleasure, sadness, anger, surprise, disgust, and fear). At the same timing, any feeling of the six basic feelings is calculated as a feeling value.

The analysis unit 202 compares the visual-point coordinates j and the coordinate values in a predetermined range of the display image h. In a case where it is determined that the visual-point coordinates j are included in coordinates in the display image h, the analysis unit 202 generates an access point (1) indicating that the gaze of the viewer 1 has accessed a region of the display image h (region previously set). In a case where it is determined that the visual-point coordinates j is not included in the coordinates in the display image h, the analysis unit 202 generates an access point (0) indicating that the gaze of the viewer 1 has not accessed the region of the display image h. The analysis unit 202 calculates the viewing point from an activation point and the access point included in the image display activation data q, by Expression (1) above.

In a manner similar to the analysis unit 102 illustrated in Fig. 2, the analysis unit 202 counts the number of access points indicating 1, calculates the count value as an instantaneous access viewer number, and calculates the instantaneous access share by Expression (4) above.

Note that, the analysis unit 202 may count the number of face images i input at the same timing, and may generate the count value as the instantaneous access viewer number. In this case, the number of visual-point coordinates j or the number of pupil diameters k input at the same timing may be counted. The count value may be generated as the instantaneous access viewer number. In a case where the number of viewers 1 is one, each of the number of face images i, the number of visual-point coordinates j, and the number of pupil diameters k input at the same timing is 1. In a case where the number of viewers 1 is n (n is an integer of two or more), each of the number of face images i, the number of visual-point coordinates j, and the number of pupil diameters k input at the same timing is n.

Processing for generating analysis results of the degree of attention and the degree of feeling by the analysis unit 202 has been known. For the detailed descriptions of the processing, refer to Patent Literature 1 above. The analysis unit 202 may acquire information on the eyeball (visual-point coordinates j and pupil diameter k) based on, for example, the eyeball image of the viewer 1, instead of the eyeball measuring device 22. In this case, the analysis unit 202 inputs, for example, the eyeball image from the eyeball measuring device 22 through the receiver 201.

(Storage unit)

The storage unit 203 stores the display image h, the face image i, the visual-point coordinates j, the pupil diameter k, the brightness data I, and the image display activation data q output by the analysis unit 202. The storage unit 203 also stores the analysis results output by the analysis unit 202. Furthermore, the storage unit 203 stores the determination results output by the determination unit 204 to be described later.

(Determination unit)

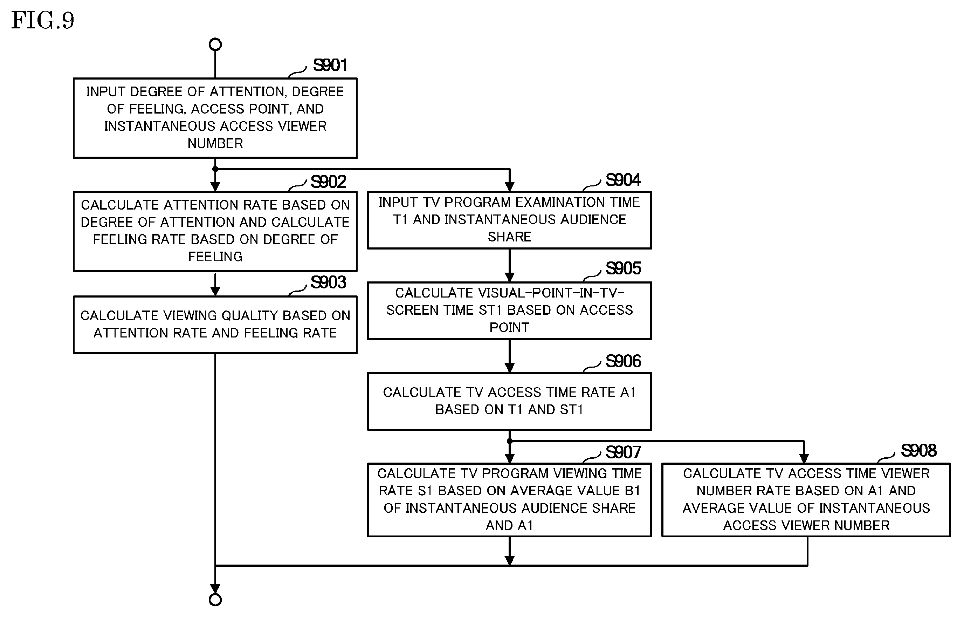

In a manner similar to the determination unit 104 illustrated in Fig. 2, the determination unit 204 inputs, for example, the degree of attention and the degree of feeling of the analysis results from the analysis unit 202, and calculates, for example, the attention rate, the feeling rate, the viewing quality, and an instantaneous visual attention rate. For example, the determination unit 204 calculates the instantaneous evaluation data of the individual viewer 1 with respect to the content of the object 2 in Fig. 15(1), the time course evaluation data of the individual viewer 1 with respect to the object 2 in Fig. 15(2), the instantaneous evaluation data of all the viewers 1 with respect to the same object 2 in Fig. 15(3), and the time course evaluation data of all the viewers 1 with respect to the same object 2 in Fig. 15(4).
Note that, the determination unit 204 may read, for example, the degree of attention and the degree of feeling of the analysis results from the storage unit 203.
First, first processing will be described as an example of processing by the determination unit 204 illustrated in Fig. 8. Fig. 9 is a flow chart of the first processing by the determination unit 204. First, the determination unit 204 inputs the degree of attention, the degree of feeling, the access point, and the instantaneous access viewer number from the analysis unit 202 (Step S901). The determination unit 204 calculates the attention rate based on the degree of attention by Expression (5) above, and calculates the feeling rate based on the degree of feeling by Expression (6) above (Step S902).
The determination unit 204 calculates the viewing quality based on the attention rate and the feeling rate that have been calculated at Step S902 by Expression (7) above (Step S903). The attention rate, the feeling rate, and the viewing quality are calculated as data in time. For example, a TV access time rate to be described later is calculated as data, including a time element, with respect to entire content of the display image h.
Like the image display 20 illustrated in Fig. 6, in a case where the object 2 to be viewed is a TV program, even when a television has been activated, the viewer 1 sometimes actually does not view the TV program. When a determination including data in which the TV program has not been viewed, is performed, a determination value with high precision cannot be acquired. As a result, content of the display image h cannot be correctly evaluated. According to the present embodiment, a determination with respect to only when the viewer 1 has viewed the TV program is performed. That is, in a case where the image display 20 is a television receiver and in a state where the television receiver has displayed a special TV program, a determination is performed based on evaluation data when a gaze of the viewer 1 has accessed the TV program. A case where the image display 20 is a television receiver will be described below.
The determination unit 204 proceeds from Step S901, and inputs TV program examination time T1 and the instantaneous audience share (Step S904). The TV program examination time T1 indicates examination time during which the TV program has been displayed. For example, the analysis unit 202 inputs response data from a response device not illustrated in Fig. 6 through the receiver 201 in accordance with an operation of an operator with respect to the response device. The analysis unit 202 calculates the TV program examination time T1 based on a timing signal included in the response data. The determination unit 204 inputs the TV program examination time T1 from the analysis unit 202.
Note that, the determination unit 204 may input the TV program examination time T1 from an external device. The TV program examination time T1 input from the external device may be stored in the storage unit 203, and the determination unit 204 may read the TV program examination time T1 from the storage unit 203. The determination unit 204 may also input the TV program examination time T1 in accordance with an operation of an operator. Furthermore, a TV program examination time measuring unit, not illustrated, included in the visual object efficacy measuring device 200, may measure the TV program examination time T1 by a known method. The determination unit 204 may input the TV program examination time T1 from the TV program examination time measuring unit.
The instantaneous audience share indicates a ratio of television receivers on which the TV program have been displayed as expressed by Expressions (2) and (3) above. The value does not depend on whether the gaze of the viewer 1 has accessed the TV program. For example, the determination unit 204 may input the instantaneous audience share from an external device. The instantaneous audience share input from the external device may be stored in the storage unit 203, and the determination unit 204 may read the instantaneous audience share from the storage unit 203. The determination unit 204 may also input the instantaneous audience share in accordance with an operation of an operator. Furthermore, an audience share measuring unit, not illustrated, included in the visual object efficacy measuring device 200, may measure the instantaneous audience share by a known method. The determination unit 204 may input the instantaneous audience share from the audience share measuring unit.

The determination unit 204 calculates a visual-point-in-TV (image)-screen time (visual-point-in-object time) ST1 based on the access point (Step S905). More specifically, the determination unit 204 counts time during which the access point indicating that the gaze of the viewer 1 has accessed the region (region previously set) of the display image h (television screen), has been input from the analysis unit 202 during the TV program examination time T1. The determination unit 204 calculates the count value as the visual-point-in-TV-screen time ST1.

The determination unit 204 calculates a TV access time rate A1 based on the TV program examination time T1 input at Step S904 and the visual-point-in-TV-screen time ST1 calculated at Step S905, by the following expression (Step S906).
[Expression 17]

$$\text{TV access time rate } A1 = \text{Visual-point-in-TV-screen time } ST1/\text{TV program examination time } T1 \ldots (17)$$

The determination unit 204 calculates a TV program viewing time rate S1 based on an average value of the instantaneous audience share B1 and the TV access time rate A1 during the TV program examination time T1, by the following expression (Step S907). The TV program viewing time rate S1 is calculated by multiplying the average value of the instantaneous audience share B1 by the TV access time rate A1, in order to evaluate all television receivers to be examined. The instantaneous audience share indicates an activation rate to all the television receivers to be examined. The TV access time rate A1 indicates a ratio in which the gaze of the viewer 1 has accessed the region of the TV program.
[Expression 18]

$$\text{TV program viewing time rate } S1 = \text{Average value of audience share } B1 \times \text{TV access time rate } A1 \ldots (18)$$

As described above, the TV program viewing time rate S1 indicates a ratio in which the gaze of the viewer 1 has actually accessed the TV program displayed on the television receiver, in all the television receivers to be examined. Therefore, the TV program can be correctly evaluated based on the TV program viewing time rate S1.

Note that, in a case where the object 2 to be viewed is not the TV program but typical content, the determination unit 204 calculates content access time rate A2 based on content examination time T2 corresponding to the TV program examination time T1, and visual-point-in-content time (visual-point-in-object time) ST2 corresponding to the visual-point-in-TV-screen time ST1, by the following expression.
[Expression 19]

$$\text{Content access time rate } A2 = \text{Visual-point-in-content time } ST2/\text{Content examination time } T2 \ldots (19)$$

The determination unit 204 calculates content viewing time rate S2 based on an average value of content instantaneous audience share B2 and the content access time rate A2, by the following expression. The content viewing time rate S2 is calculated by multiplying the average value of the content instantaneous audience share B2 by the content access time rate A2, in order to evaluate all terminals to be examined. The content instantaneous audience share indicates an activation rate with respect to all the terminals to be examined. The content access time rate A2 indicates a ratio in which the gaze of the viewer 1 has accessed a region of the content.
[Expression 20]

Content viewing time rate S2 = Average value of content instantaneous audience share B2 ×

Content access time rate A2 ... (20)

The determination unit 204 proceeds from Step S906, and averages the instantaneous access viewer number input at Step S901 with the TV program examination time T1. The determination unit 204 calculates a TV access time viewer number rate based on the TV access time rate A1 calculated at Step S906 and the average value of the instantaneous access viewer number, by the following expression (Step S908).

[Expression 21]

TV access time viewer number rate = TV access time rate A1 × Average value of instantaneous

access viewer number ... (21)

Typically, the number of viewers 1 with respect to a TV is often more than one. For example, a family often views the TV. As described above, the eyeball measuring device 22 irradiates infrared rays from the infrared irradiation LED 24 to the eye of the viewer 1. The eyeball measuring device 22 captures a line of cornea reflection so as to measure the visual-point coordinates j of the viewer 1. In a case where the number of viewers 1 is more than one, visual-point coordinates j of each of the viewers 1 are measured. Similarly, a pupil diameter k and a face image i captured by the face capturing device 21 are measured for each of the viewers. Therefore, the analysis unit 202 calculates the instantaneous access viewer number based on the number of visual-point coordinates j, the number of pupil diameters k, or the number of pupil diameters k input at the same timing. The determination unit 204 calculates the TV access time viewer number rate in Expression (21) above using an average value of the instantaneous access viewer number.

As describe above, the TV access time rate A1 is a value in a case where one viewer 1 is estimated independent of the number of viewers 1 who have accessed. In a case where the number of viewers 1 who have accessed is more than one, an access share is estimated to increase by the number of viewers 1. The access share is calculated as the TV access time viewer number rate. The TV access time viewer number rate may exceed 100%. The TV access time viewer number rate can be calculated in time when a TV program has been viewed. The TV access time viewer number rate can be calculated as an average value through the entire TV program. Therefore, the TV program that has been broadcast can be correctly evaluated based on the TV access time viewer number rate.

The TV program broadcast can be correctly evaluated using four determination values including the average value of the instantaneous audience share B1, the TV access time rate A1, the TV program viewing time rate, and the TV access time viewer number rate. Which determination value is used depends on a purpose of evaluation.

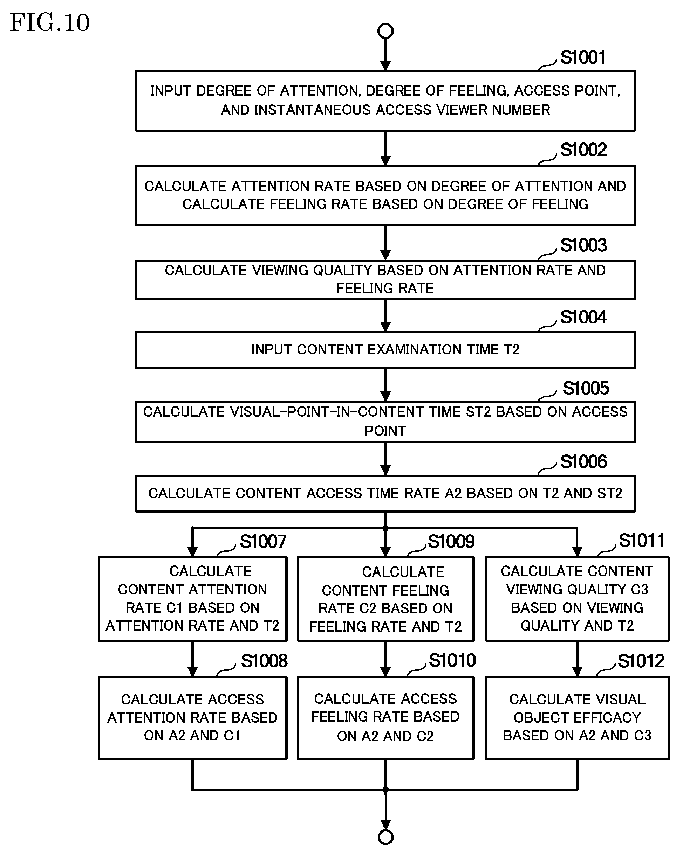

Next, second processing will be described as an example of the other processing by the determination unit 204 illustrated in Fig. 8. Fig. 10 is a flow chart of the second processing by the determination unit 204. First, the determination unit 204 inputs the degree of attention, the degree of feeling, the access point, and the instantaneous access viewer number from the analysis unit 202 (Step S1001). The determination unit 204 calculates the attention rate (%) based on the degree of attention and the feeling rate (%) based on the degree of feeling by Expressions (5) and (6) above, respectively (Step S1002).

The determination unit 204 calculates the viewing quality based on the attention rate and the feeling rate calculated at Step S1002 by Expression (7) above (Step S1003). A case where the object 2 to be viewed is content such as the display image h displayed on the image display 20 will be described below.

The determination unit 204 inputs the content examination time T2 (Step S1004). The content examination time T2 indicates evaluation object time during which the viewer 1 has viewed the content. A method, by which the determination unit 204 inputs the content examination time T2, is the same as the method by which the determination unit 204 inputs the TV program examination time T1 as described at Step S904 in Fig. 9.

The determination unit 204 calculates the visual-point-in-content time ST2 based on the access point (Step S1005). More specifically, the determination unit 204 counts time during which the access point indicating that the gaze of the viewer 1 has accessed the content, has been input from the analysis unit 202 during the content examination time T2. The determination unit 204 calculates the count value as the visual-point-in-content time ST2.

The determination unit 204 calculates the content access time rate A2 based on the content examination time T2 input at Step S1004 and the visual-point-in-content time ST2 calculated at Step S1005, by Expression (19) above (Step S1006).

As described above, the content access time rate A2 indicates a ratio in which the gaze of the viewer 1 has accessed a region of the content in a state where the image display 20 has displayed the content. Therefore, the content to be viewed can be correctly evaluated based on the content access time rate A2.

The determination unit 204 calculates a content attention rate (content average attention rate) C1 of the entire content based on the attention rate (content instantaneous attention rate) calculated at Step S1002 and the content examination time T2 input at Step S1004, by the following expression (Step S1007).
[Expression 22]

$$\text{Content attention rate (content average attention rate)}\,C1 = \frac{\int_{t=0}^{t=T2}\text{Content instantaneous attention rate }dt}{\text{Content examination time }T2}$$

... (22)

As described above, the content attention rate C1 indicates an average value of the attention rate with respect to the content. Therefore, the content can be correctly evaluated during the content examination time T2 based on the content attention rate C1.
The determination unit 204 calculates an access attention rate of the content including an access share with respect to the content based on the content access time rate A2 calculated at Step S1006 and the content attention rate C1 calculated at Step S1007, by the following expression (Step S1008). A maximum value of the access attention rate is 100%.
[Expression 23]

Access attention rate of content = Content access time rate A2× Content attention rate

C1/100 ... (23)

As described above, the access attention rate of the content indicates an attention rate including time during which the gaze of the viewer 1 has actually accessed the content. Therefore, the content can be correctly evaluated based on the access attention rate.
The determination unit 204 proceeds from Step S1006, and calculates a content feeling rate of the entire content (content average feeling rate) C2 based on the feeling rate (content instantaneous feeling rate) calculated at Step S1002 and the content examination time T2 input at Step S1004, by the following expression (Step S1009).
[Expression 24]

$$\text{Content feeling rate (content average feeling rate)}\,C2 = \frac{\int_{t=0}^{t=T2}\text{Content instantaneous feeling rate }dt}{\text{Content examination time }T2}$$

... (24)

As described above, the content feeling rate C2 indicates an average value of the feeling rate with respect to the content. Therefore, the content can be correctly evaluated based on the content feeling rate C2 during the content examination time T2.
The determination unit 204 calculates an access feeling rate of the content including the access share with respect to the content, based on the content access time rate A2 calculated at Step S1006 and the content feeling rate C2 calculated at Step S1009, by the following expression (Step S1010). A maximum value of the access feeling rate is 100%.
[Expression 25]

Access feeling rate of content = Content access time rate A2 × Content feeling rate C2/100 ...

(25)

As described above, the access feeling rate of the content indicates a feeling rate including time during which the gaze of the viewer 1 has actually accessed the content. Therefore, the content can be correctly evaluated based on the access feeling rate.

The determination unit 204 proceeds from Step S1006, and calculates a content viewing quality of the entire content (content average viewing quality) C3 based on the viewing quality calculated at Step S1003 (content instantaneous viewing quality) and the content examination time T2 input at Step S1004, by the following expression (Step S1011).

[Expression 26]

$$\text{Content viewing quality (content average viewing quality)} \, C3 = \frac{\int_{t=0}^{t=T2} \text{Content instantaneous viewing quality } dt}{\text{Content examination time T2}} \quad \dots (26)$$

As described above, the content viewing quality C3 indicates an average value of the viewing quality with respect to the content. Therefore, the content can be correctly evaluated based on the content viewing quality C3 during the content examination time T2.

The determination unit 204 calculates visual object efficacy of the content including the access share with respect to the content, based on the content access time rate A2 calculated at Step S1006 and the content viewing quality C3 calculated at Step S1011, by the following expression (Step S1012). A maximum value of the visual object efficacy is 100%.

[Expression 27]

Visual object efficacy of content = Content access time rate A2 × Content viewing quality

C3/100 ... (27)

As described above, the visual object efficacy of the content indicates a viewing quality including time during which the gaze of the viewer 1 has actually accessed the content. Therefore, the content can be correctly evaluated based on the visual object efficacy.

(Display unit)

Referring back to Fig. 8, the display unit 205 inputs the determination results, such as the visual object efficacy, from the determination unit 204. The display unit 205 displays, for example, the determination results on a display device, such as a display.

(Communication unit)

The communication unit 206 reads data, such as the display image h and the face image i, the analysis results, and the determination results from the storage unit 203. The communication unit 206 transmits these pieces of data to the data-collection-and-statistics center 31 through the network 30.

As described above, according to the visual object efficacy measuring device 200 of the second embodiment of the present invention, the determination unit 204 has been made to calculate the viewing quality in which the emotion-and-feeling including the emotion and the feeling combined has been converted into a numerical form, based on the degree of attention and the degree of feeling with respect to the display image h displayed on the image display 20. The determination unit 204 calculates the visual-point-in-TV-screen time ST1 indicating time during which the gaze of the viewer 1 has accessed to the region of the display image h, based on the access point indicating whether the gaze of the viewer 1 has accessed the region of the display image h. The determination unit 204 calculates the TV access time rate A1 based on the TV program examination time T1 and the visual-point-in-TV-screen time ST1. The determination unit 204 calculates the TV program viewing time rate S1 based on the TV access time rate A1 and the average value of the instantaneous audience share B1. The determination unit 204 calculates the TV access time viewer number rate based on the TV access time rate A1 and the average value of the instantaneous access viewer number indicating the number of viewers 1 who have accessed the region of the display image h.

The determination unit 204 calculates the visual-point-in-content time ST2 indicating time during which the gaze of the viewer 1 has accessed the region of the content, based on the access point. The determination unit 204 calculates the content access time rate A2 based on the content examination time T2 and the visual-point-in-content time ST2. The determination unit 204 calculates the content attention rate C1, based on the attention rate and the content examination time T2. The determination unit 204 calculates the access attention rate, based on the content access time rate A2 and the content attention rate C1. The determination unit 204 calculates the content feeling rate C2, based on the feeling rate and the content examination time T2. The determination unit 204 calculates the access feeling rate, based on the content access time rate A2 and the content feeling rate C2. The determination unit 204 calculates the content viewing quality C3, based on the viewing quality and the content examination time T2. The determination unit 204 calculates the visual object efficacy, based on the content access time rate A2 and the content viewing quality C3.

Accordingly, the TV program viewing time rate S1 that is an audience share taking a ratio in which the viewer 1 has actually accessed, into account, and the TV access time rate A1 taking the number of viewers 1 who have accessed, into account, are calculated with respect to the display image h displayed on the image display 20. The content attention rate C1 that is an attention rate, the content feeling rate C2 that is a feeling rate, and the content viewing quality C3 that is a viewing quality, are calculated with respect to the content during the content examination time T2 that is examination time. In a state where the content has been displayed on the image display 20, the access attention rate that is an attention rate, and the access feeling rate that is a feeling rate are calculated, the attention rate and the feeling rate taking time during the viewer 1 has accessed, into account. With respect to the content during the content examination time T2 that is evaluation object time, the viewing quality taking the time during the viewer 1 has accessed, into account, is calculated as the visual object efficacy in Expression (27) above.

These pieces of data, such as the TV program viewing time rate S1, can be displayed as determination values for evaluating the content, such as the display image h. Therefore, the content, such as the display image, viewed by the viewer 1 can be further correctly evaluated.

For example, in a case where the object 2 is a TV program, conventionally, the evaluation is performed based on only a ratio of the number of image displays 20 on which the TV program has been displayed (activation rate), the ratio being the instantaneous audience share. According to the visual object efficacy measuring device 200 of the second embodiment of the present invention, the TV program has been made to be evaluated based on the visual object efficacy in Expression (27) above. Thus, the TV program viewing time rate S1, and the viewing quality including the degree of attention and the degree of feeling, can be added. Therefore, further correct evaluation can be achieved.

In a case where the object 2 is a TV CM, conventionally, the evaluation is also performed with only a ratio of the number of image displays 20 on which a TV program has been displayed (activation rate), the ratio being the instantaneous audience share. Evaluation for the CM receives an effect due to, for example, content of a program just before the CM, or a field of interest of the viewer 1 at the point in time. Therefore, it is difficult to evaluate the CM, correctly. According to the visual object efficacy measuring device 200 of the second embodiment of the present invention, the CM has been made to be evaluated based on the visual object efficacy in Expression (27) above. Thus, for example, the content viewing time rate S2 and the viewing quality including the degree of attention and the degree of feeling, with respect to the CM, can be added. Furthermore, the degree of impact of the viewer 1 with respect to the CM itself can be determined. Therefore, further correct evaluation can be achieved including various effects, such as content of a program just before and a field of interest of the viewer 1 at the point in time.

The present invention has been described with two embodiments. The present invention is not limited to the above two embodiments. Various alternations may be made without departing from the scope of the technical idea of the invention. For example, in the above embodiment, the determination unit 104 has been made to calculate the feeling rate, based on the degree of feeling of the six basic feelings, by Expression (6) above. The determination unit 104 has been made to calculate the viewing quality, using the feeling rate of the six basic feelings, by Expression (7) above. The determination unit has been made to calculate the instantaneous visual object efficacy, using the viewing quality calculated based on the feeling rate of the six basic feelings, by Expression (10) above. In contrast, the determination unit 104 may focus on a special feeling previously set in the six basic feelings in accordance with a purpose of evaluation. The determination unit 104 may calculate a feeling rate, based on the degree of feeling of the special feeling, by Expression (6) above. The determination unit 104 may calculate a viewing quality, using the feeling rate of the special feeling, by Expression (7). The determination unit 104 may calculate instantaneous visual object efficacy, using the viewing quality of the special feeling, by Expression (10) above. For example, in a case where only the degree of feeling of pleasure in the six basic feelings (the degree of positive feeling) is focused, the determination unit 104 calculates a positive feeling rate, based on the degree of positive feeling, by Expression (6) above. The determination unit 104 calculates a positive viewing quality, based on the positive feeling rate, by Expression (7) above. The determination unit calculates positive instantaneous visual object efficacy, using the positive viewing quality calculated based on the positive feeling rate, by Expression (10) above. Similarly, instantaneous visual object efficacy in Expression (15) above and visual object efficacy in Expression (16) above are calculated.

The same is true of the determination unit 204 according to the above second embodiment. That is, the determination unit 204 focuses on a special feeling previously set in the six basic feelings. The determination unit 204 calculates a feeling rate, based on the degree of feeling of the feeling. The determination unit 204 calculates visual object efficacy, using a viewing quality of the special feeling, by Expression (27) above.

The processing of the analysis unit 202 of the visual object efficacy measuring device 200 according to the above second embodiment may be added to the analysis unit 102 of the visual object efficacy measuring device 100 according to the above embodiment. The processing of the determination unit 204 of the visual object efficacy measuring device 200 may be added to the determination unit 104 of the visual object efficacy measuring device 100.

The visual object efficacy measuring devices 100 and 200 may be a stationary device or a portable device. The visual object efficacy measuring device 200, the image display 20, the face capturing device 21, the eyeball measuring device 22, and the brightness measuring device 23 may be integrally included in a portable device.

Next, examples of applications of the visual object efficacy measuring device 100 according to the embodiment of the present invention and the visual object efficacy measuring device 200 according to the second embodiment of the present invention will be described. Examples of applications of rating of the object 2, selection of video content, excitement determination of video content, measurement of the degree of possibility of purchase with respect to a commodity will be described below.

(Rating of object 2)

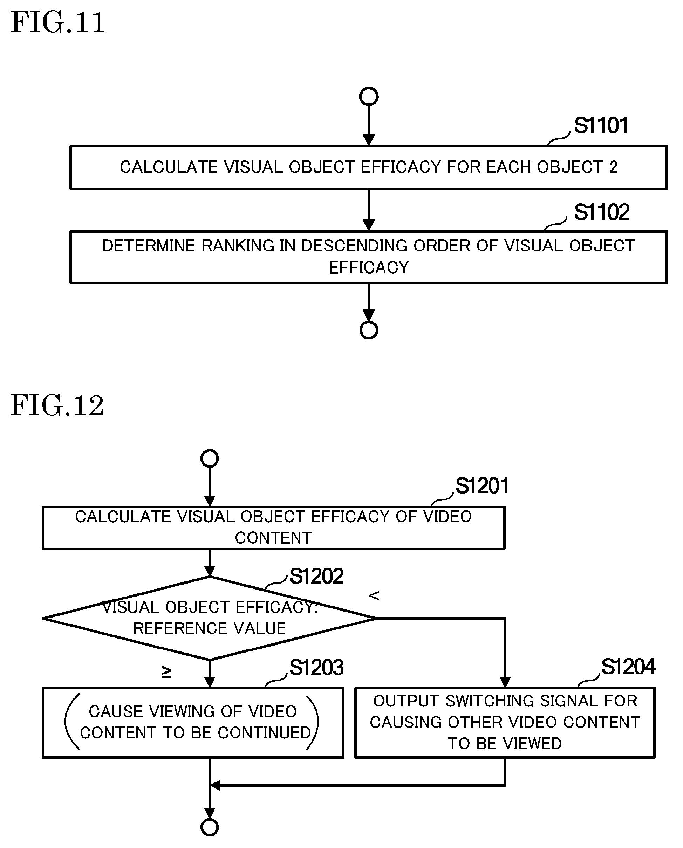

First, an example of having applied the visual object efficacy measuring device 100 according to the embodiment of the present invention to rating of the object 2 will be described. Fig. 11 is a flow chart of processing of the determination unit 104 in the example of rating the object 2. In each of types of object 2 (category), the determination unit 104 calculates visual object efficacy for each of a plurality of objects 2 included in the type, by the processing illustrated in Fig. 3 (for example, instantaneous visual object efficacy in Expression (10) above) (Step S1101). The determination unit 104 determines ranking of the objects 2 in descending order of values of the pieces of visual object efficacy. The determination unit 104 generates the visual object efficacy and the ranking for each of the objects 2 as determination results (Step S1102). Examples of the types of objects 2 include video content, an electronic book, a display board, and a commodity.

Accordingly, the rating can be performed to the plurality of objects 2 belonging to the same type. Each of the plurality of objects 2 belonging to the same type can be correctly evaluated. For example, ranking can be achieved in descending order of impacts of film works or TV programs, in order of efficacy of CMs, or in order of best-selling predictions of commodities.

Note that, the determination unit 104 may focus on a special feeling in the six basic feelings (for example, only a feeling of pleasure). The determination unit 104 may calculate visual object efficacy (positive visual object efficacy) using the degree of feeling of the special feeling (for example, the degree of positive feeling) so as to determine ranking of the objects 2.

Similarly, the visual object efficacy measuring device 200 according to the second embodiment of the present invention can be applied to rating of content of the display image h.

(Selection of video content)

Next, an example of having applied the visual object efficacy measuring device 100 according to the embodiment of the present invention to selection of video content will be described. The object 2 is defined as the video content. The visual object efficacy measuring device 100 includes a video content selecting unit not illustrated in Fig. 2 and a video content display unit not illustrated in Fig. 2. The video content selecting unit selects a piece of video content from a plurality of pieces of video content. The video content display unit displays the video content on a screen. Fig. 12 is a flow chart of processing of the determination unit 104 in an example of selecting the video content.

The video content selecting unit selects one piece of video content from a plurality of pieces of video content by an operation of an operator or an instruction from the determination unit 104, and displays the one pieces of video content on the video content display unit. Accordingly, the viewer 1 views the one piece of video content from the plurality of pieces of video content. The receiver 101 receives a visual image a of the video content displayed on the video content display unit.

First, the video content selecting unit selects one piece of video content from a plurality of pieces of video content (video content X) by an operation of an operator, and displays the video content X on the video content display unit. Accordingly, the viewer 1 views the video content X. The receiver 101 receives a visual image a of the video content X displayed on the video content display unit.

In accordance with the processing illustrated in Fig. 3, the determination unit 104 calculates visual object efficacy of the video content X (for example, instantaneous visual object efficacy in Expression (10) above) when the viewer 1 has viewed the video content X during a period of time, namely, when predetermined access time has passed (Step S1201). The determination unit 104 compares the visual object efficacy of the video content X and a reference value previously set (Step S1202). In a case where the visual object efficacy is the reference value or more, the determination unit 104 causes the viewer 1 to continue to view the video content X (Step S1203). Note that, this comparison processing has been made to determine whether the visual object efficacy is the reference value or more. The comparison processing may be made to determine whether the visual object efficacy is larger than the reference value. The following comparison processing may be made to determine whether a value is a predetermined value or more, or whether the value is larger than the predetermined value. The following comparison processing may be made to determine whether the value is the predetermined value or less, or whether the value is smaller than the predetermined value.

Meanwhile, the determination unit 104 causes the viewer 1 to view another piece of video content in a case where the visual object efficacy is lower than the reference value. In this case, the determination unit 104 outputs a switching signal to the video content selecting unit (Step S1204). The video content selecting unit selects video content Y that has not selected from the plurality of pieces of video content, and displays the video content Y on the video content display unit. Accordingly, the viewer 1 views the video content Y. The receiver 101 receives a visual image a of the video content Y displayed on the video content display unit.

In a manner similar to the above video content X, the determination unit 104 calculates visual object efficacy of the video content Y, and compares the visual object efficacy and the reference value previously set. In a case where the visual object efficacy is the reference value or more, the determination unit 104 causes the viewer 1 to continue to view the video content Y. In a case where the visual object efficacy is less than the reference value, the determination unit 104 causes the viewer 1 to view another piece of video content. The determination unit 104 outputs, as determination results, the visual object efficacy of the video content, identifying information of the video content (information for identifying, for example, the pieces of video content X and Y), and a comparison result to the reference value.

As described above, the determination unit 104 repeats the processing illustrated in Fig. 3 with different pieces of video content until the visual object efficacy of the video content becomes the reference value or more. Accordingly, the video content can be correctly evaluated. Viewer's 1 favorite video content can be automatically selected. The viewer 1 can sequentially view different pieces of video content until the determination unit 104 specifies a piece of video content with visual object efficacy that is the reference value or more. Finally, the viewer 1 can continue to view the piece of video content with the visual object efficacy that is the reference value or more. The above selecting method of video content is effective in a case where video content suitable to sensitivity of the viewer 1 is searched.

Note that, the determination unit 104 may focus on a special feeling in the six basic feelings (for example, only a feeling of pleasure in a case where a viewer's 1 favorite is comedy content), and calculate visual object efficacy (positive visual object efficacy) using the degree of feeling of the special feeling (for example, the degree of positive feeling). The determination unit 104 may compare the visual object efficacy and the reference value, and select video content with the visual object efficacy higher than the reference value. The determination unit 104 may cause the viewer 1 to continue to view the video content.

Similarly, the visual object efficacy measuring device 200 according to the second embodiment of the present invention can be applied to the selection of video content.

(Excitement determination of video content)

Next, an example of having applied the visual object efficacy measuring device 100 according to the embodiment of the present invention to excitement determination of video content will be described. The object 2 is defined as the video content. Typically, in a case where the viewer 1 views the video content in a state where the degree of attention is a predetermined value (for example, a level of 0.5) or more and the degree of feeling of each of the six basic feelings (pleasure, sadness, anger, surprise, disgust, and fear) is a predetermined value (for example, a level of 3) or more, it can be determined that the viewer 1 has paid attention to the video content and viewed the video content while causing the degree of feeling to be high. In a case where the viewer 1 has viewed the video content in a state where the degree of attention is lower than a predetermined value (for example, a level of 0), it can be determined that the viewer 1 has viewed the video content while being bored.

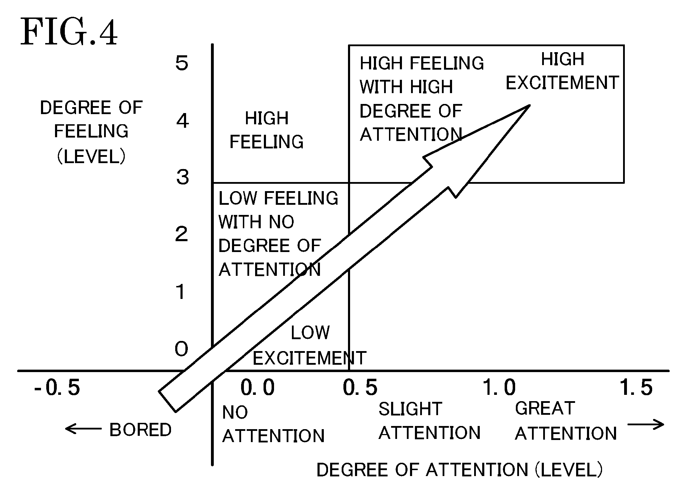

Fig. 4 is a graphical representation for describing the degree of excitement with respect to the video content. As illustrated in Fig. 4, when the viewer 1 has viewed the video content, the higher a level of the degree of attention is and the higher a level of the degree of feeling is, it can be determined that the more excited the viewer 1 has been.

For example, the larger a ratio of time during which the degree of attention of the viewer 1 is a predetermined value (for example, a level of 0.5) or more and the degree of feeling is a predetermined value (for example, a level of 3) or more, to entire viewing time of the video content, is, it can be determined that the more excited the viewer 1 has been at the video content.

In Expression (5) above, when the degree of attention > 0.5 is defined, the attention rate > 25 is satisfied. In Expression (6) above, when the degree of feeling > 3.0 is defined, the feeling rate > 60 is satisfied. Here, the attention rate = 25 and the feeling rate = 60 are defined, in Expression (7) above, Viewing quality = {k1 x Attention rate (%)} x {k2 x Feeling rate (%)}/100 = 15 x k1 x k2 is satisfied. In the example of the present application, the visual object efficacy to be calculated (instantaneous visual object efficacy in Expression (15) above) and visual object efficacy in a case where the viewing quality = (15 x k1 x k2) is defined, are compared. As a ratio between time, during which the first visual object efficacy has been larger than the second visual object efficacy, and predetermined examination time is larger, it can be determined that the viewer 1 has been more excited at the video content. The detailed descriptions will be given below.

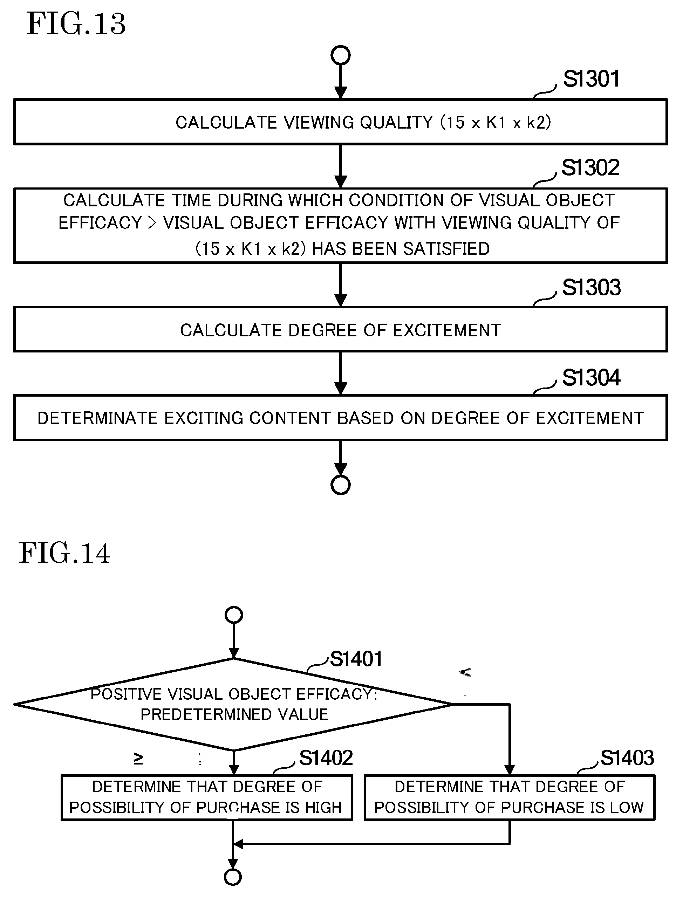

Fig. 13 is a flow chart of processing of the determination unit 104 in an example of the degree of excitement with respect to the video content. In addition to the processing illustrated in Fig. 3, the determination unit 104 calculates the viewing quality (15 x k1 x k2) to be a reference, by multiplying a weighting coefficient k1 of the attention rate, a weighting coefficient k2 of the feeling rate, and a predetermined value (for example, 15) (Step S1301). The determination unit 104 compares the visual object efficacy that has been calculated (instantaneous visual object efficacy in Expression (15) above) and the visual object efficacy in a case of the viewing quality to be a reference. During access time, the determination unit 104 calculates time during which a condition has been satisfied, the condition in which the visual object efficacy > the visual object efficacy when the viewing quality is (15 x k1 x k2) has been satisfied (Step S1302). The larger a ratio of the calculated time to the access time is, the determination unit 104 determines that the more excited the viewer 1 has been at the video content. That is, the determination unit 104 calculates the degree of excitement with respect to the video content by the following expression (Step S1303).

[Expression 28]

$$\text{Degree of excitement} = \frac{\int_0^{\text{Access time}} t \, (\text{time during which visual object efficacy} > \text{visual object efficacy with viewing quality of } (15 \times k1 \times k2) \text{ has been satisfied}) \, dt}{\text{Access time}}$$

... (28)

The determination unit 104 determines that the video content is content at which the viewer 1 becomes excited in a case where the degree of excitement calculated by Expression (28) above is larger than a predetermined value. In a case where the degree of excitement is the predetermined value or less, the determination unit 104 determines that the video content is content at which the viewer 1 does not become excited (Step S1304). The determination unit 104 outputs, as determination results, the visual object efficacy of the video content, the degree of excitement, and a result indicating whether the content is exciting.

Accordingly, it can be determined whether the content of the video content is content at which the viewer 1 becomes excited. The video content can be correctly evaluated.

Similarly, the visual object efficacy measuring device 200 according to the second embodiment of the present invention can be applied to the excitement determination of video content.

(Measurement of the degree of possibility of purchase with respect to commodity)

Next, an example of having applied the visual object efficacy measuring device 100 of the embodiment of the present invention to measurement of the degree of possibility of purchase with respect to a commodity will be described. The object 2 is defined as goods, such as a commodity. Typically, in a case where the viewer 1 has viewed the commodity in a state where the degree of attention is lower than a predetermined value, the commodity is considered to be not conspicuous. Thus, it can be determined that the degree of possibility of purchase is low.

In contrast, in a case where the viewer 1 has viewed the commodity in a state where the degree of attention is a predetermined value (for example, a level of 0.5) or more, attention has been paid to the commodity. However, it cannot be necessarily determined that the degree of possibility of purchase is high. In this case, in a case where the viewer 1 has viewed the commodity in a state where the degree of feeling of sadness, angler, disgust, or fear in the six basic feelings is a predetermined value (for example, a level of 3) or more, it can be determined that the degree of refusal of purchase is high. That is, in a case where the viewer 1 has viewed the commodity in a state where the degree of attention is a predetermined value (for example, a level of 0.5) or more (in a state where the commodity is conspicuous) and in a state where the degree of feeling of pleasure in the six basic feelings (the degree of positive feeling) is a predetermined value or more, it can be determined that the degree of possibility of purchase is high.

Fig. 5 is a graphical representation for describing the degree of possibility of purchase with respect to the commodity. As illustrated in Fig. 5, when the viewer 1 has viewed the commodity, as a level of the degree of attention is higher and a level of the degree of positive feeling is higher, it can be determined that the degree of possibility of purchase is higher with respect to the commodity.

More specifically, the degree of feeling is defined as the degree of feeling of pleasure in the six basic feelings (the degree of positive feeling) in Expression (2) above. The determination unit 104 calculates a positive feeling rate. The feeling rate is defined as the positive feeling rate in Expression (6) above. The determination unit 104 calculates a positive viewing quality. The viewing quality is defined as the positive viewing quality in Expression (7) above. The determination unit 104 calculates positive visual object efficacy.

For example, in Expression (5) above, when the degree of attention > 0.5 is defined, the attention rate > 25 is satisfied. In Expression (6) above, when the degree of positive feeling > 3.0 is defined, the positive feeling rate > 60 is satisfied. Here, the attention rate = 25 and the positive feeling rate = 60 are defined, Positive viewing quality = {k1 x Attention rate (%)} x {k2 x Positive feeling rate(%)}/100 = 15 x k1 x k2 is satisfied in Expression (7) above.

Fig. 14 is a flow chart of processing of the determination unit 104 in the example of measuring the degree of possibility of purchase with respect to the commodity. The determination unit 104 compares the positive visual object efficacy (instantaneous visual object efficacy in Expression (15) above) and a predetermined value (for example, positive visual object efficacy in a case where the positive viewing quality is defined as (15 x k1 x k2) (Step S1401). In a case where the positive visual object efficacy is larger than the predetermined value, it is determined that the degree of possibility of purchase is high (Step S1402). In a case where the positive visual object efficacy is the predetermined value or less, it is determined that the degree of possibility of purchase is low (Step S1403). In this case, the determination unit 104 outputs, as determination results, the positive visual object efficacy of the commodity, and a result indicating whether the degree of possibility of purchase is high.

Similarly, the visual object efficacy measuring device 200 according to the second embodiment of the present invention can be applied to the measurement of the degree of possibility of purchase with respect to a commodity in the display image h.

Note that, a typical computer can be used for a hardware configuration of each of the visual object efficacy measuring device 100 according to the embodiment of the present invention and the visual object efficacy measuring device 200 according to the second embodiment of the present invention. Each of the visual object efficacy measuring devices 100 and 200 includes a computer having volatile storage media, such as a CPU and a RAM, a non-volatile storage medium, such as a ROM, and an interface. Individual functions of the receiver 101, the analysis unit 102, the determination unit 104, and the display unit 105 included in the visual object efficacy measuring device 100 are achieved by causing the CPU to execute a program including the functions written therein. Individual functions of the receiver 201, the analysis unit 202, the determination unit 204, the display unit 205, and the communication unit 206 included in the visual object efficacy measuring device 200 are also achieved by causing the CPU to execute a program including the functions written therein. Each of these programs is stored in the above storage medium. Each of the programs is read and executed by the CPU. In addition, each of these programs can be stored in a storage medium, such as a magnetic disk (for example, a floppy (registered trademark) disk, or a hard disk), an optical disk (for example, a CD-ROM or a DVD), or a semiconductor memory, can be distributed, and can be transmitted/received through a network.

The entire disclosure of the description, the claims, the abstract, and the drawings included in Japanese Patent Application No. 2013-216642, filed October 17, 2013, is incorporated herein by reference.

Industrial Applicability

A visual object efficacy measuring device according to the present invention is useful in various fields including measurement of efficacy for evaluating an object, such as content to be viewed by a human.

Reference Sings List

| 1 | viewer |
| 2 | object |
| 3 | eye camera |
| 4 | eyeball capturing device |
| 5 | visual scene capturing device |
| 6 | microphone |
| 7 | transmitter |
| 8 | storage device |
| 9 | body movement capturing device |
| 10 | body temperature measuring device |
| 11, 23 | brightness measuring device |
| 12 | response device |
| 13 | terminal to be examined |
| 20 | image display |
| 21 | face capturing device |
| 22 | eyeball measuring device |
| 24 | infrared irradiation LED |
| 25 | tracking lens |
| 26 | pupil-diameter measuring lens |
| 30 | network |
| 31 | data-collection-and-statistics center |

| 100, 200 | visual object efficacy measuring device |
|---|---|
| 101, 201 | receiver |
| 102, 202 | analysis unit |
| 103, 203 | storage unit |
| 104, 204 | determination unit |
| 105, 205 | display unit |
| 206 | communication unit |

**Claims**

1. A visual object efficacy measuring device comprising:

   an analysis unit configured to acquire a pupil diameter from an eyeball image of at least one viewer viewing at least one object displayed on at least one terminal to be examined, calculate the degree of attention with respect to the at least one object based on the pupil diameter of the at least one viewer, acquire a predetermined part of a face of the at least one viewer from a face image of the at least one viewer, and calculate the degree of feeling with respect to the at least one object based on a movement of the predetermined part of the face of the at least one viewer; and
   a determination unit configured to calculate visual object efficacy as an evaluation value for evaluating the at least one object, based on the degree of attention and the degree of feeling calculated by the analysis unit,

   wherein the determination unit calculates a viewing quality by multiplying the degree of attention and the degree of feeling calculated by the analysis unit, calculates a viewing rate by multiplying an audience share and an access share, the audience share indicating a ratio of the number of the at least one terminal to be examined on which the at least one object has been displayed to the total number of the at least one terminal to be examined, the access share indicating a ratio of the number of the at least one viewer whose gaze has accessed the at least one object to the total number of the at least one viewer, multiplies the viewing quality and the viewing rate, and defines a result acquired by the multiplication as the first of the visual object efficacy.

2. The visual object efficacy measuring device according to claim 1,
   wherein the analysis unit further acquires visual-point coordinates indicating a position of the visual point of the at least one viewer, from the eyeball image of the at least one viewer, and generates access information indicating whether the gaze of the at least one viewer has accessed the at least one object, based on the visual-point coordinates, and
   the determination unit further calculates, as visual-point-in-object time, time during which the gaze of the at least one viewer has accessed the at least one object, based on the access information generated by the analysis unit, calculates an access time rate, based on predetermined examination time and the visual-point-in-object time, calculates a viewing time rate by multiplying the audience share and the access time rate, and defines the viewing time rate as the second of the visual object efficacy.

3. The visual object efficacy measuring device according to claim 1,
   wherein the analysis unit further acquires visual-point coordinates indicating a position of the visual point of the at least one viewer, from the eyeball image of the at least one viewer, generates access information indicating whether the gaze of the at least one viewer has accessed the at least one object, based on the visual-point coordinates, and calculates, as an access viewer number, the number of the at least one viewer that has accessed the at least one object, based on the eyeball image or the face image, and
   the determination unit further calculates as visual-point-in-object time, time during which the gaze of the at least one viewer has accessed the at least one object, based on the access information generated by the analysis unit, calculates an access time rate, based on predetermined examination time and the visual-point-in-object time, calculates an access time viewer number rate by multiplying the access time rate and the access viewer number calculated by the analysis unit, and defines the access time viewer number rate as the third of the visual object efficacy.

4. The visual object efficacy measuring device according to claim 1,
   wherein the determination unit calculates pieces of the visual object efficacy for a plurality of the objects included in the same category, and the pieces of the visual object efficacy rates in descending order.

5. The visual object efficacy measuring device according to claim 1,

wherein the at least one object is defined as video content, and

the determination unit calculates the visual object efficacy of the video content, compares the visual object efficacy and a predetermined reference value,

in a case where the visual object efficacy is lower than the reference value, the determination unit causes the at least one viewer to view other video content, calculates and compares the visual object efficacy of the other video content to the reference value, performs the calculation and comparison processing until the determination unit selects video content in which the visual object efficacy becomes the reference value or more, and causes the at least one viewer to view different video content during a period of time until the visual object efficacy becomes the reference value or more.

6. The visual object efficacy measuring device according to claim 1,

wherein the at least one object is defined as video content, and

the determination unit further compares the visual object efficacy and a predetermined value, calculates, as the degree of excitement, a ratio of time during which the visual object efficacy has been larger than the predetermined value to predetermined evaluation object time during which the at least one viewer has accessed the at least one object,

in a case where the degree of excitement is larger than a predetermined threshold value, the determination unit determines that the video content is content at which the at least one viewer becomes excited, and

in a case where the degree of excitement is the predetermined threshold value or less, the determination unit determines that the video content is content at which the at least one viewer does not become excited.

7. The visual object efficacy measuring device according to claim 1,

wherein the at least one object is defined as a commodity, and

the determination unit calculates a positive viewing quality by multiplying the degree of attention and the degree of feeling of a feeling of pleasure, with respect to the at least one object, calculated by the analysis unit, multiplies the positive viewing quality and the viewing rate, and defines a result acquired by the multiplication as positive visual object efficacy,

in a case where the positive visual object efficacy is larger than a predetermined threshold value, the determination unit determines that the commodity is goods in which the degree of possibility of purchase is high, and

in a case where the positive visual object efficacy is the predetermined threshold value or less, the determination unit determines that the commodity is goods in which the degree of possibility of purchase is low.

# FIG.1

VISUAL OBJECT EFFICACY
MEASURING DEVICE 100

BODY
MOVEMENT
IMAGE e

BODY MOVEMENT
CAPTURING DEVICE 9

BODY
TEMPERATURE
MEASURING 10
DEVICE

BODY
TEMPERATURE
DATA f

VISUAL IMAGE a
EYEBALL MOVEMENT
IMAGE b
VOICE DATA c

RESPONSE DATA d

BRIGHTNESS DATA g

TERMINAL-TO-BE-
EXAMINED
ACTIVATION DATA s

11    BRIGHTNESS
MEASURING DEVICE

TERMINAL TO
BE EXAMINED 13

EYE CAMERA 3

VISUAL SCENE
CAPTURING DEVICE 5

GAZE

2 OBJECT

EYEBALL CAPTURING 4
DEVICE

MICROPHONE 6

RESPONSE 12
DEVICE

TRANSMITTER 7

STORAGE 8
DEVICE

VIEWER 1

# FIG.2

VISUAL OBJECT EFFICACY MEASURING DEVICE 100

SYNCHRONIZING SIGNAL DATA
+
a~g, s

VISUAL IMAGE a

EYEBALL MOVEMENT IMAGE b

VOICE DATA c c

RESPONSE DATA d

BODY MOVEMENT IMAGE e

BODY TEMPERATURE DATA f

BRIGHTNESS DATA g

TERMINAL-TO-BE-EXAMINED ACTIVATION DATA s

101 RECEIVER

102 ANALYSIS UNIT

103 STORAGE UNIT

104 DETERMINATION UNIT

105 DISPLAY UNIT

ANALYSIS RESULTS

ANALYSIS RESULTS

DETERMINATION RESULTS

EP 3 058 873 A1

# FIG.3

**S301**
INPUT ANALYSIS RESULTS
(FOR EXAMPLE, DEGREE OF ATTENTION AND DEGREE OF FEELING)

**S302**
CALCULATE ATTENTION RATE BASED ON DEGREE OF ATTENTION AND
CALCULATE FEELING RATE BASED ON DEGREE OF FEELING

**S303**
CALCULATE VIEWING QUALITY BASED ON ATTENTION RATE AND
FEELING RATE

**S304**
CALCULATE INSTANTANEOUS VISUAL ATTENTION RATE BASED ON
VIEWING POINT AND ATTENTION RATE

**S305**
CALCULATE INSTANTANEOUS VISUAL FEELING RATE BASED ON
VIEWING POINT AND FEELING RATE

**S306**
CALCULATE INSTANTANEOUS VISUAL OBJECT EFFICACY BASED ON
VIEWING POINT AND VIEWING QUALITY

**S307**
OUTPUT, FOR EXAMPLE, INSTANTANEOUS VISUAL OBJECT EFFICACY

# FIG.4

DEGREE OF FEELING (LEVEL)

5
4
3
2
1
0

HIGH FEELING WITH HIGH DEGREE OF ATTENTION

HIGH EXCITEMENT

HIGH FEELING

LOW FEELING WITH NO DEGREE OF ATTENTION

LOW EXCITEMENT

-0.5    0.0    0.5    1.0    1.5

← BORED    NO ATTENTION    SLIGHT ATTENTION    GREAT ATTENTION    →

DEGREE OF ATTENTION (LEVEL)

# FIG.5

DEGREE OF
POSITIVE FEELING
(LEVEL)

5

HIGH
DEGREE OF
POSITIVE
FEELING

4

HIGH DEGREE OF
POSSIBILITY OF PURCHASE

3

ALMOST NO DEGREE
OF POSSIBILITY OF
PURCHASE DUE TO
INCONSPICUOUSNESS

2

LOW DEGREE
OF POSSIBILITY
OF PURCHASE

1

0

LOW DEGREE OF
POSITIVE FEELING

-0.5      0.0      0.5      1.0      1.5

← NO DEGREE        NO          SLIGHT        GREAT
OF ATTENTION    ATTENTION    ATTENTION    ATTENTION →

DEGREE OF
ATTENTION (LEVEL)

# FIG.6

DATA-COLLECTION-
AND-STATISTICS
CENTER        31

NETWORK        30

VISUAL OBJECT
EFFICACY MEASURING
DEVICE  200

IMAGE DISPLAY 20、
FACE CAPTURING DEVICE 21、
EYEBALL MEASURING DEVICE 22、
BRIGHTNESS MEASURING DEVICE 23

GAZE

DISPLAY IMAGE h、
FACE IMAGE i、
VISUAL-POINT COORDINATES j、
PUPIL DIAMETER k、
BRIGHTNESS DATA l、
IMAGE DISPLAY ACTIVATION DATA q

VIEWER1

# FIG.7

VISUAL OBJECT EFFICACY
MEASURING DEVICE  200

(NETWORK  30)

(*1)(*2)(*3)(*4) →

FACE CAPTURING
DEVICE  21

BRIGHTNESS
MEASURING DEVICE  23

IMAGE DISPLAY  20

(*2)

FACE IMAGE  i

(*4)

BRIGHTNESS
DATA l

(*1)

DISPLAY IMAGE h、
IMAGE DISPLAY
ACTIVATION DATA q

(*3)

VISUAL-POINT
COORDINATES  j
PUPIL DIAMETER k

EYEBALL MEASURING
DEVICE  22

TRACKING LENS  25-1

INFRARED IRRADIATION
LED  24

PUPIL-DIAMETER MEASURING
LENS  26

TRACKING LENS  25-2

EP 3 058 873 A1

# FIG.8

VISUAL OBJECT EFFICACY
MEASURING DEVICE 200

SYNCHRONIZING
SIGNAL DATA
+
h~l, q

DISPLAY IMAGE h

FACE IMAGE i

VISUAL-POINT
COORDINATES j

PUPIL DIAMETER k

BRIGHTNESS DATA l

IMAGE DISPLAY
ACTIVATION DATA q

201 RECEIVER

203 STORAGE UNIT

206 COMMUNICATION UNIT

NETWORK 30

202 ANALYSIS UNIT

ANALYSIS RESULTS

204 DETERMINATION UNIT

ANALYSIS RESULTS

DETERMINATION RESULTS

205 DISPLAY UNIT

EP 3 058 873 A1

**FIG.9**

S901 — INPUT DEGREE OF ATTENTION, DEGREE OF FEELING, ACCESS POINT, AND INSTANTANEOUS ACCESS VIEWER NUMBER

S902 — CALCULATE ATTENTION RATE BASED ON DEGREE OF ATTENTION AND CALCULATE FEELING RATE BASED ON DEGREE OF FEELING

S903 — CALCULATE VIEWING QUALITY BASED ON ATTENTION RATE AND FEELING RATE

S904 — INPUT TV PROGRAM EXAMINATION TIME T1 AND INSTANTANEOUS AUDIENCE SHARE

S905 — CALCULATE VISUAL-POINT-IN-TV-SCREEN TIME ST1 BASED ON ACCESS POINT

S906 — CALCULATE TV ACCESS TIME RATE A1 BASED ON T1 AND ST1

S907 — CALCULATE TV PROGRAM VIEWING TIME RATE S1 BASED ON AVERAGE VALUE B1 OF INSTANTANEOUS AUDIENCE SHARE AND A1

S908 — CALCULATE TV ACCESS TIME VIEWER NUMBER RATE BASED ON A1 AND AVERAGE VALUE OF INSTANTANEOUS ACCESS VIEWER NUMBER

## FIG.10

```
                                    ○
                                    │
                                    ▼              ╭─S1001
        ┌───────────────────────────────────────────────────┐
        │ INPUT DEGREE OF ATTENTION, DEGREE OF FEELING,      │
        │ ACCESS POINT, AND INSTANTANEOUS ACCESS VIEWER      │
        │ NUMBER                                             │
        └───────────────────────────────────────────────────┘
                                    │
                                    ▼              ╭─S1002
        ┌───────────────────────────────────────────────────┐
        │ CALCULATE ATTENTION RATE BASED ON DEGREE OF        │
        │ ATTENTION AND CALCULATE FEELING RATE BASED ON      │
        │ DEGREE OF FEELING                                  │
        └───────────────────────────────────────────────────┘
                                    │
                                    ▼              ╭─S1003
        ┌───────────────────────────────────────────────────┐
        │ CALCULATE VIEWING QUALITY BASED ON ATTENTION       │
        │ RATE AND FEELING RATE                              │
        └───────────────────────────────────────────────────┘
                                    │
                                    ▼              ╭─S1004
        ┌───────────────────────────────────────────────────┐
        │ INPUT CONTENT EXAMINATION TIME T2                  │
        └───────────────────────────────────────────────────┘
                                    │
                                    ▼              ╭─S1005
        ┌───────────────────────────────────────────────────┐
        │ CALCULATE VISUAL-POINT-IN-CONTENT TIME ST2 BASED   │
        │ ON ACCESS POINT                                    │
        └───────────────────────────────────────────────────┘
                                    │
                                    ▼              ╭─S1006
        ┌───────────────────────────────────────────────────┐
        │ CALCULATE CONTENT ACCESS TIME RATE A2 BASED ON T2  │
        │ AND ST2                                            │
        └───────────────────────────────────────────────────┘
```

| S1007 | S1009 | S1011 |
|---|---|---|
| CALCULATE CONTENT ATTENTION RATE C1 BASED ON ATTENTION RATE AND T2 | CALCULATE CONTENT FEELING RATE C2 BASED ON FEELING RATE AND T2 | CALCULATE CONTENT VIEWING QUALITY C3 BASED ON VIEWING QUALITY AND T2 |
| ▼ S1008 | ▼ S1010 | ▼ S1012 |
| CALCULATE ACCESS ATTENTION RATE BASED ON A2 AND C1 | CALCULATE ACCESS FEELING RATE BASED ON A2 AND C2 | CALCULATE VISUAL OBJECT EFFICACY BASED ON A2 AND C3 |

# FIG.11

CALCULATE VISUAL OBJECT EFFICACY FOR EACH OBJECT 2 _S1101_

DETERMINE RANKING IN DESCENDING ORDER OF VISUAL OBJECT EFFICACY _S1102_

# FIG.12

CALCULATE VISUAL OBJECT EFFICACY OF VIDEO CONTENT _S1201_

VISUAL OBJECT EFFICACY: REFERENCE VALUE _S1202_

<

≥

CAUSE VIEWING OF VIDEO CONTENT TO BE CONTINUED _S1203_

OUTPUT SWITCHING SIGNAL FOR CAUSING OTHER VIDEO CONTENT TO BE VIEWED _S1204_

## FIG.13

```
                                    ○
                                    │
                                    ▼                          S1301
┌─────────────────────────────────────────────────────────────────┐
│           CALCULATE VIEWING QUALITY (15 x K1 x k2)                │
└─────────────────────────────────────────────────────────────────┘
                                    │
                                    ▼                          S1302
┌─────────────────────────────────────────────────────────────────┐
│        CALCULATE TIME DURING WHICH CONDITION OF VISUAL OBJECT     │
│        EFFICACY > VISUAL OBJECT EFFICACY WITH VIEWING QUALITY OF  │
│              (15 x K1 x k2) HAS BEEN SATISFIED                    │
└─────────────────────────────────────────────────────────────────┘
                                    │
                                    ▼                          S1303
┌─────────────────────────────────────────────────────────────────┐
│               CALCULATE DEGREE OF EXCITEMENT                      │
└─────────────────────────────────────────────────────────────────┘
                                    │
                                    ▼                          S1304
┌─────────────────────────────────────────────────────────────────┐
│   DETERMINATE EXCITING CONTENT BASED ON DEGREE OF EXCITEMENT      │
└─────────────────────────────────────────────────────────────────┘
                                    │
                                    ▼
                                    ○
```

## FIG.14

```
                        ○
                        │
                        ▼                S1401
              ◇─────────────────────◇        <
              POSITIVE VISUAL OBJECT EFFICACY :
                 PREDETERMINED VALUE
              ◇─────────────────────◇
                ≥                    │
                │         S1402      │              S1403
┌───────────────────────────────┐ ┌───────────────────────────────┐
│    DETERMINE THAT DEGREE OF    │ │    DETERMINE THAT DEGREE OF    │
│  POSSIBILITY OF PURCHASE IS    │ │  POSSIBILITY OF PURCHASE IS    │
│             HIGH               │ │             LOW                │
└───────────────────────────────┘ └───────────────────────────────┘
                │                                 │
                ▼◄────────────────────────────────┘
                ○
```

FIG.15

```
┌─────────────────────┐                    ┌─────────────────────┐
│ (1) INSTANTANEOUS   │   TIME COURSE      │ (2) TIME COURSE     │
│ EVALUATION DATA OF  │ ─────────────────► │ EVALUATION DATA OF  │
│ INDIVIDUAL VIEWER 1 │                    │ INDIVIDUAL VIEWER 1 │
│ WITH RESPECT TO     │                    │ WITH RESPECT TO     │
│ OBJECT 2            │                    │ OBJECT 2            │
└─────────────────────┘                    └─────────────────────┘
         │  FOR EXAMPLE,                              │  FOR EXAMPLE,
         │  AVERAGING                                 │  AVERAGING
         ▼         TIME COURSE,                       ▼
┌─────────────────────┐    AND,            ┌─────────────────────┐
│ (3) INSTANTANEOUS   │ FOR EXAMPLE,       │ (4) TIME COURSE     │
│ EVALUATION DATA OF  │  AVERAGING         │ EVALUATION DATA OF  │
│ ALL VIEWERS 1 WITH  │ ─────────────────► │ ALL VIEWERS 1 WITH  │
│ RESPECT TO SAME     │                    │ RESPECT TO SAME     │
│ OBJECT 2            │                    │ OBJECT 2            │
└─────────────────────┘                    └─────────────────────┘
```

FIG.16

ACTIVATION POINT X ACCESS POINT

ACTIVATION POINT (TERMINAL ACTIVATION/NO TERMINAL ACTIVATION)

VIEWING POINT (VIEWING/NO VIEWING)

ACCESS POINT (ACCESS/NO ACCESS)

VIEWING POINT x VIEWING QUALITY

VIEWING POINT x ATTENTION RATE

INSTANTANEOUS VISUAL ATTENTION RATE

INSTANTANEOUS VISUAL OBJECT EFFICACY

INSTANTANEOUS VISUAL FEELING RATE

VIEWING POINT x FEELING RATE

ATTENTION RATE

VIEWING QUALITY

FEELING RATE

ATTENTION RATE x FEELING RATE

EP 3 058 873 A1

FIG.17

# FIG.18

A : NUMBER OF TERMINALS TO BE EXAMINED 13

B : NUMBER OF TERMINALS TO BE EXAMINED 13 (ACTIVATION)
= NUMBER OF VIEWERS 1

C : NUMBER OF VIEWERS 1 (ACCESS)

G : ATTENTION RATE
H : FEELING RATE

# FIG.19

B : NUMBER OF TELEVISIONS
(TV PROGRAM DISPLAY)
= NUMBER OF VIEWERS 1

C : NUMBER OF VIEWERS
(ACCESS TO TV PROGRAM)

G : ATTENTION RATE
H : FEELING RATE

# FIG.20

A : NUMBER OF, FOR EXAMPLE, PCs TO BE
    EXAMINED
B : NUMBER OF, FOR EXAMPLE, PCs
    (FOR EXAMPLE, CM DISPLAY)
        = NUMBER OF VIEWERS 1

C : NUMBER OF VIEWERS 1
(FOR EXAMPLE, ACCESS TO CM)

G : ATTENTION RATE

H : FEELING RATE

# FIG.21

A : NUMBER OF TERMINALS TO BE EXAMINED

B : NUMBER OF TERMINALS
(WEB SCREEN DISPLAY)
    = NUMBER OF VIEWERS 1

C : NUMBER OF VIEWERS 1
(ACCESS TO WEB SCREEN)

G : ATTENTION RATE

H : FEELING RATE

# FIG.22

A : NUMBER OF PIECES OF PRINTED MATTER INTRODUCED

B : NUMBER OF PIECES OF PRINTED MATTER PURCHASED
= NUMBER OF VIEWERS 1

C : NUMBER OF VIEWERS 1 (ACCESS TO PRINTED    MATTER)

G : ATTENTION RATE

H : FEELING RATE

# FIG.23

A : NUMBER OF VISITORS

B : NUMBER OF VISITORS HAVING PASSED IN FRONT OF DISPLAY BOARD
= NUMBER OF VIEWERS 1

C : NUMBER OF VISITORS (VIEWERS 1) HAVING VIEWED DISPLAY BOARD

G : ATTENTION RATE

H : FEELING RATE

# FIG.24

A : NUMBER OF VISITORS

B : NUMBER OF VISITORS HAVING
PASSED IN FRONT OF COMMODITY
= NUMBER OF VIEWERS 1

C : NUMBER OF VISITORS
(VIEWERS 1) HAVING VIEWED
COMMODITY

G : ATTENTION RATE

H : FEELING RATE

## INTERNATIONAL SEARCH REPORT

| | International application No. |
|---|---|
| | PCT/JP2014/077559 |

### A. CLASSIFICATION OF SUBJECT MATTER
*A61B5/16*(2006.01)i, *A61B3/11*(2006.01)n

According to International Patent Classification (IPC) or to both national classification and IPC

### B. FIELDS SEARCHED

Minimum documentation searched (classification system followed by classification symbols)
A61B5/16, A61B3/11

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| A | Wataru KURASHIMA, Koichi KIKUCHI, Kazutoshi UEDA, "Dokokei Hanno to Kao Hyojo Hanno no Yugo ni yoru Jokan Hyoka no Atarashii Hoho", Eizo Joho Industrial, vol.43, no.6, whole no.807, Kohei WAKEBE, 01 June 2011 (01.06.2011), pages 24 to 28 | 1-7 |
| A | WO 2011/042989 A1 (Koichi KIKUCHI), 14 April 2011 (14.04.2011), entire text; all drawings (Family: none) | 1-7 |
| A | JP 2011-139441 A (Panasonic Corp.), 14 July 2011 (14.07.2011), entire text; all drawings & US 2011/0209066 A1 | 1-7 |

☐ Further documents are listed in the continuation of Box C.     ☐ See patent family annex.

| | |
|---|---|
| *  Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 09 February 2015 (09.02.15) | 24 February 2015 (24.02.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011042989 A

- JP 2013216642 A